(19) Europäisches Patentamt
European Patent Office
Office européen des brevets

(11) EP 4 471 017 A1

(12) EUROPEAN PATENT APPLICATION
published in accordance with Art. 153(4) EPC

(43) Date of publication:
**04.12.2024 Bulletin 2024/49**

(21) Application number: **23746276.7**

(22) Date of filing: **19.01.2023**

(51) International Patent Classification (IPC):
***C07D 311/36** (2006.01)* ***A61K 31/352** (2006.01)*
***A61P 31/14** (2006.01)*

(52) Cooperative Patent Classification (CPC):
**A61K 31/352; A61P 31/14; A61P 31/16; C07D 311/36**

(86) International application number:
**PCT/CN2023/073234**

(87) International publication number:
**WO 2023/143397 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.01.2022 CN 202210107552**

(71) Applicants:
- **Shanghai Institute of Materia Medica, Chinese Academy of Sciences**
  **Shanghai 201203 (CN)**
- **Institute of Chinese Materia Medica, China Academy of Chinese Medical Sciences**
  **Beijing 100007 (CN)**

(72) Inventors:
- **HUANG, Chenggang**
  **Shanghai 201203 (CN)**
- **CUI, Xiaolan**
  **Beijing 100007 (CN)**
- **XIE, Yang**
  **Shanghai 201203 (CN)**
- **TIAN, Xiaoting**
  **Shanghai 201203 (CN)**
- **GUO, Shanshan**
  **Beijing 100007 (CN)**
- **LI, Zhixiong**
  **Shanghai 201203 (CN)**
- **GAO, Yingjie**
  **Beijing 100007 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) CYCLIC(ALKYL)(AMINO)ALKOXY-SUBSTITUTED ARYL-PYRONE COMPOUND AND USE THEREOF

(57) Disclosed in the present invention are a novel compound represented by general formula I, a pharmaceutically acceptable salt thereof, a solvate, a stereoisomer including mixtures thereof in all ratios, and a pharmaceutical composition containing the compound, wherein $R_{1a}$, $R_{1b}$, $R_{1c}$, $R_2$, $R_{3a}$, $R_{3b}$, $R_{4a}$, $R_{4b}$, $R_5$, $R_6$, $X_1$, $X_2$, $X_3$, ring A, etc. have the meanings given in the description. The present invention also relates to a preparation method for the compound, and also relates to a use of the compound, the pharmaceutically acceptable salt thereof, the solvate, and the stereoisomer including the mixtures in all ratios, and in particular to a use of the compound in the preparation of antiviral drugs..

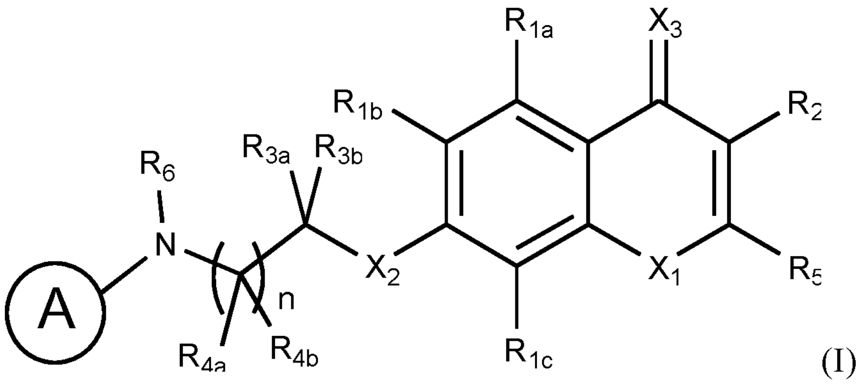

(I)

## Description

## Technical field

The present invention provides a new compound and pharmaceutically acceptable salts, solvates, stereoisomers including mixtures in various proportions thereof, and pharmaceutical compositions containing said compound. The present invention also relates to the use of such compounds and pharmaceutically acceptable salts, solvates, stereoisomers including mixtures in various proportions thereof, particularly in the preparation of antiviral drugs.

## Background

A series of antiviral drugs have been developed in this field, such as the drugs against influenza virus, Baloxavir (Baloxavir marboxil), Paramivir (Peramivir), Laninamivir (Laninamivir octanoate), Oseltamivir, (Oseltamivir phosphate), Zanamivir ( Zanamivir), amantadine (Rimantadine), amantadine (Amantadine), etc. However, there are no effective drugs against respiratory viruses such as parainfluenza virus, respiratory syncytial virus, adenovirus, coronavirus, etc., and broad-spectrum antiviral drugs are needed, such as ribavirin, or interferon, etc. Meanwhile, lopinavir/ritonavir, chloroquine phosphate, arbidol, darunavir, and hydroxychloroquine, favipiravir, remdesivir, and other drugs are commonly used for new coronavirus pneumonia. These antiviral drugs still have many shortcomings in terms of therapeutic efficacy and safety of use, therefore, there is still a need to develop novel antiviral therapeutic drugs in this field.

## Summary of the Invention

It is an object of the present invention to provide an isoflavonoid derivative and a pharmaceutically acceptable salt thereof.

The first aspect of the present invention provides a compound shown in formula I, or a pharmaceutically acceptable salt, solvate, optically pure isomer, stereoisomer, or mixture thereof.

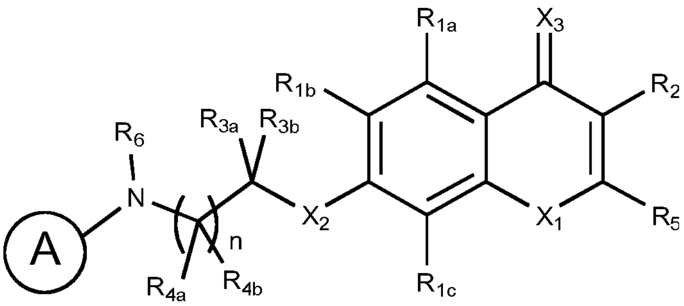

(I)

wherein $X_1$, $X_2$ are each independently selected from the group consisting of: O, $NR_7$; wherein said $R_7$ is selected from the group consisting of: a hydrogen, alkyl, substituted alkyl, arylalkyl, substituted arylalkyl, cycloalkyl, substituted cycloalkyl;

$X_3$ is selected from the following group: O, $NR_8$; wherein said $R_8$ is selected from the following group: hydrogen, alkyl;
$R_{1a}$, $R_{1b}$, $R_{1c}$ are each independently selected from the following group: a hydrogen, hydroxyl, alkoxy;
$R_2$ is selected from the following group: a substituted or unsubstituted aryl, or substituted or unsubstituted heteroaryl;
$R_{3a}$, $R_{3b}$ are each independently selected from the following group: a hydrogen, alkyl, halogen;
$R_{4a}$, $R_{4b}$ are each independently selected from the following group: a hydrogen, alkyl, halogen;
$R_5$ is selected from the following group: a hydrogen or alkoxy;
$R_6$ is selected from the following group: a hydrogen, substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, substituted or unsubstituted alkoxycarbonyl, substituted or unsubstituted carbamoyl, or $(CH_2)_tR_7$; wherein t is 1, 2, 3, 4, 5 or 6; wherein said $R_7$ is selected from the following group: a $C_2$-$C_6$ acyl, $C_2$-$C_6$ amido, aryl, heteroaryl, cycloalkyl, heterocycloalkyl, $NR_8COR_9$; Said $R_8$ is selected from the following group: a hydrogen, alkyl; $R_9$ is selected from the following group: an alkyl, aryl, heteroaryl, cycloalkyl, heterocycloalkyl;

The A ring is a substituted or unsubstituted cycloalkyl;
n is 1, 2, 3, 4, 5, 6, 7, 8, 9 or 10;

Wherein each "substituted" means that a hydrogen atom on a group is replaced by one or more substituents selected from the group consisting of: a cyano, halogen, alkyl, hydroxy, alkoxy, alkenyl, alkynyl, aryl, heteroaryl; and, said compound of formula I does not comprise

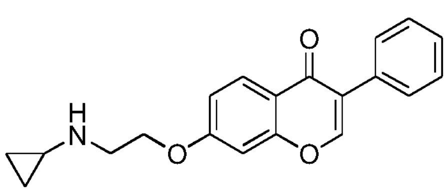
,
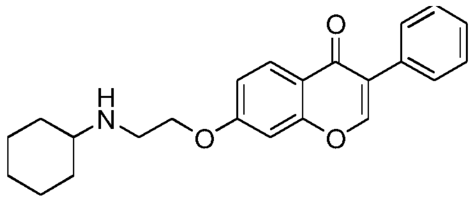
;

In the above formulae, unless otherwise specified, the alkyl group is a $C_1$-$C_6$ alkyl group, the aryl group is a $C_6$-$C_{10}$ aryl group, the cycloalkyl group is a $C_3$-$C_8$ cycloalkyl group, the alkoxy group is a $C_1$-$C_6$ alkoxy group, the alkenyl group is a $C_2$-$C_6$ alkenyl group, the alkynyl group is a $C_2$-$C_6$ alkynyl group, and the heteroaryl group is a heteroaryl group of 5-12 members (more preferably, 5-7 members).

In another preferred example, $X_1$ is O; and/or $X_2$ is O.

In another preferred example, said $R_7$ is selected from the following group: a hydrogen, alkyl.

In another preferred example, $X_3$ is O.

In another preferred example, $R_{1a}$, $R_{1b}$, $R_{1c}$ are each independently selected from a hydrogen.

In another preferred example, $R_2$ is a substituted or unsubstituted aryl, preferably a substituted or unsubstituted phenyl.

In another preferred example, $R_{3a}$, $R_{3b}$ are each independently selected from the following group: a hydrogen, $C_1$-$C_4$ alkyl, halogen.

In another preferred example, $R_{4a}$, $R_{4b}$ are each independently selected from the following group: a hydrogen, $C_1$-$C_4$ alkyl, halogen.

In another preferred example, $R_5$ is selected from a hydrogen or $C_1$-$C_4$ alkoxy.

In another preferred example, $R_6$ is selected from the following group: a hydrogen, substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, or $(CH_2)_tR_7$; wherein t is 1, 2, 3, 4, 5 or 6; $R_7$ is selected from the following group: a $C_2$-$C_6$ acyl, $C_2$-$C_6$ amido, $NR_8COR_9$; Said $R_8$ is selected from the following group: a hydrogen, alkyl; $R_9$ is selected from the following group: an alkyl, aryl.

In another preferred example, the A ring is a substituted or unsubstituted $C_3$-$C_7$ cycloalkyl, preferably a $C_3$-$C_6$ cycloalkyl.

In another preferred example, n is 1, 2, 3 or 4.

In another preferred example, said $R_6$ is selected from the group consisting of: a hydrogen, substituted or unsubstituted alkyl.

In another preferred example, said $R_6$ is selected from the group consisting of: a hydrogen, unsubstituted alkyl.

In another preferred example, said compound of formula I has the structure shown in formula II,

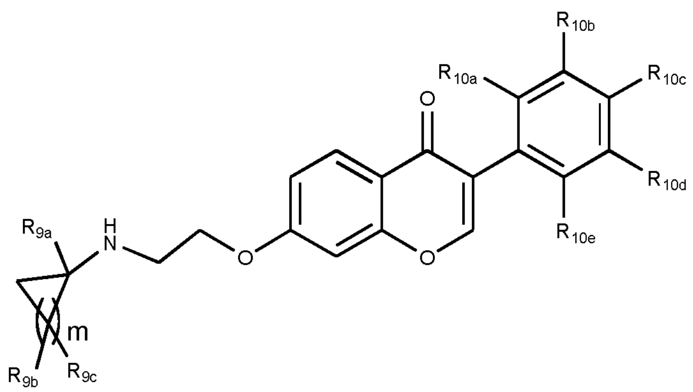

(II)

wherein $R_{9a}$, $R_{9b}$, $R_{9c}$ are each independently selected from the group consisting of: a hydrogen, hydroxyl, halogen, cyano, $C_1$-$C_6$ alkyl, $C_2$-$C_6$ alkenyl, $C_2$-$C_6$ alkynyl, $C_6$-$C_{10}$ aryl, 5-12 membered heteroaryl;

$R_{10a}$, $R_{10b}$, $R_{10c}$, $R_{10d}$, $R_{10e}$ are each independently selected from the group consisting of: a hydrogen, hydroxyl, $C_1$-$C_6$ alkoxy, halogen, cyano;
m is 1, 2, 3, 4 or 5.

In another preferred example, the compound of formula II is selected from the following group:

| | |
|---|---|
| 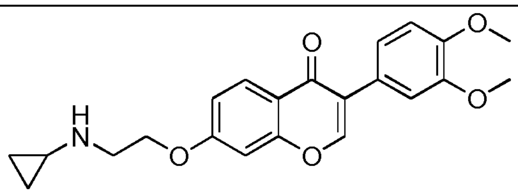 Compound 1 | 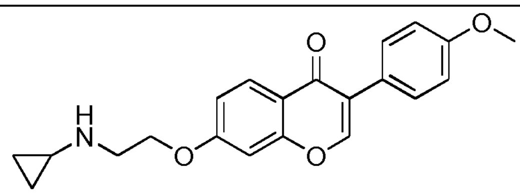 Compound 2 |
| 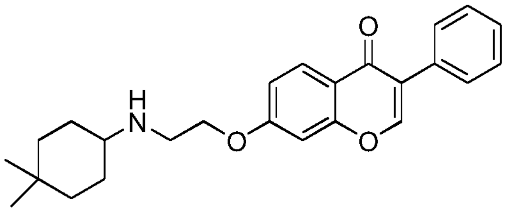 Compound 3 | 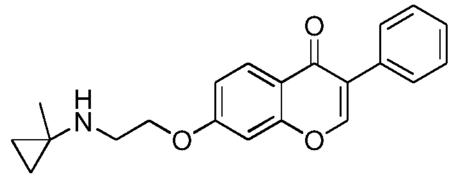 Compound 5 |
| 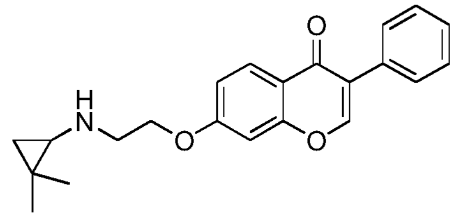 Compound 6 | 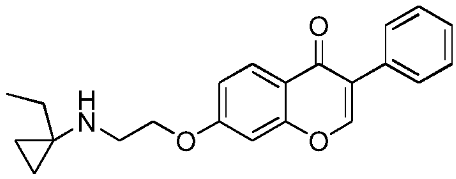 Compound 8 |

In another preferred example, said compound of formula I has the structure shown in formula III,

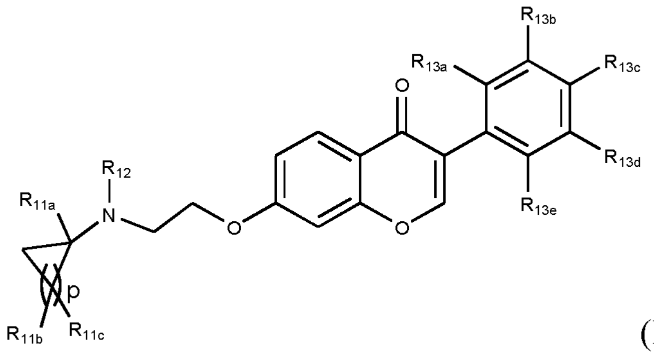

(III)

$R_{11a}$, $R_{11b}$, $R_{11c}$ are each independently selected from the group consisting of: a hydrogen, hydroxyl, halogen, cyano, C1-C6 alkyl, C2-C6 alkenyl, C2-C6 alkynyl, C1-C6 alkoxy C6-C10 aryl, and 5-12 memdered heteroaryl;
$R_{12}$ is selected from the following group: a substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, substituted or unsubstituted alkoxycarbonyl, substituted or unsubstituted carbamoyl, or $(CH_2)_tR_7$; wherein t is 1, 2, 3, 4, 5, or 6; wherein said $R_7$ is selected from the group consisting of: a $C_2$-$C_6$ acyl, $C_2$-$C_6$ amido, aryl, heteroaryl, cycloalkyl, heterocycloalkyl, $NR_8COR_9$; wherein said $R_8$ is selected from the group consisting of: a hydrogen, alkyl; and $R_9$ is selected from the group consisting of: an alkyl, aryl, heteroaryl, cycloalkyl, heterocycloalkyl;
$R_{13a}$, $R_{13b}$, $R_{13c}$, $R_{13d}$, $R_{13e}$ are each independently selected from the group consisting of: a hydrogen, hydroxyl, alkoxy, halogen, cyano.

In another preferred example, the compound of formula III is selected from the following group:

| | |
|---|---|
| 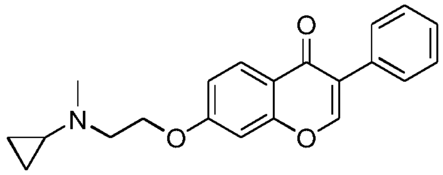 Compound 9 | 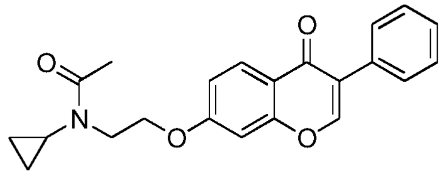 Compound 10 |
| 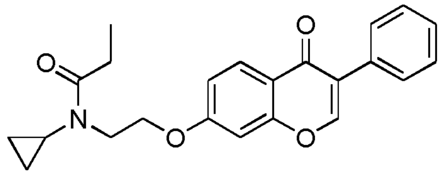 Compound 11 | 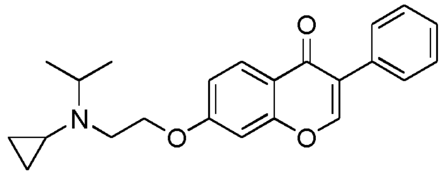 Compound 12 |
| 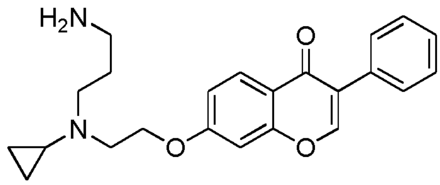 Compound 13 | 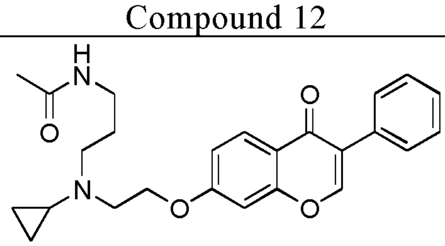 Compound 14 |
| 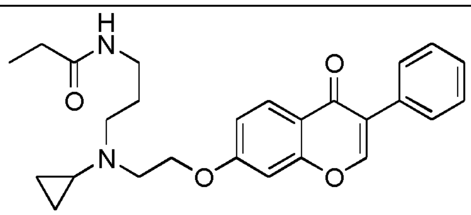 Compound 15 | 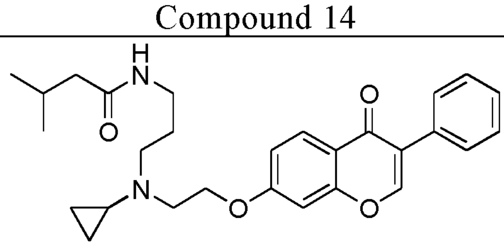 Compound 16 |
| 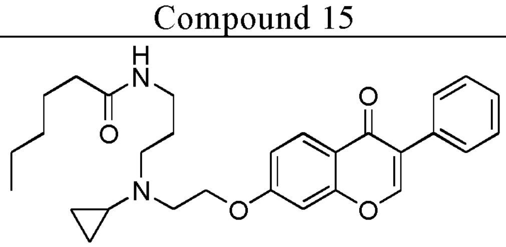 Compound 17 | 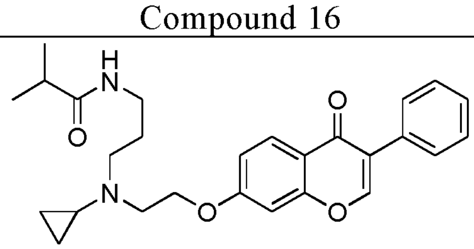 Compound 18 |

| | |
|---|---|
| 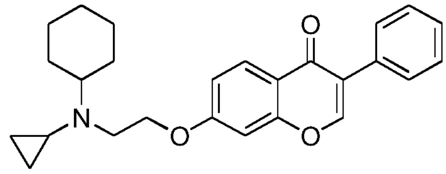<br>Compound 19 | 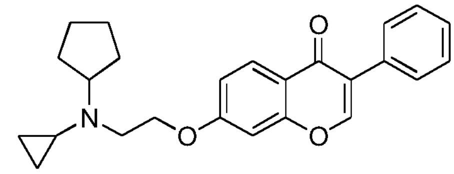<br>Compound 20 |
| 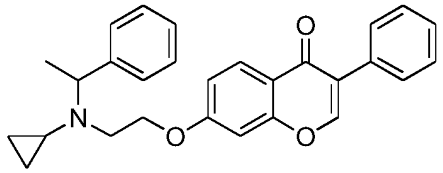<br>Compound 21 | 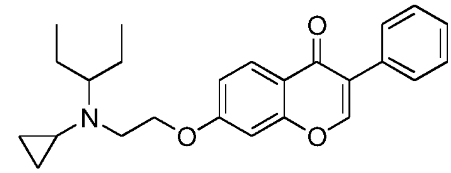<br>Compound 22 |

A second aspect of the present invention provides a pharmaceutical composition, said pharmaceutical composition comprising the compound described in the first aspect,

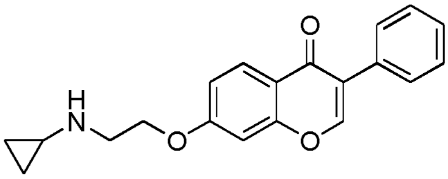
or
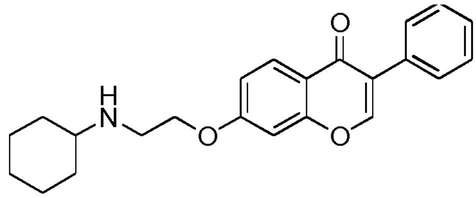
,

or a pharmaceutically acceptable salt, solvate, optically pure isomer or stereoisomer thereof; and a pharmaceutically acceptable carrier.

In a third aspect of the present invention, there is provided a use of a compound of formula I, or a pharmaceutically acceptable salt, solvate, optically pure isomer, stereoisomer, or mixture thereof, a pharmaceutical composition described in the second aspect of the present invention for the preparation of a pharmaceutical composition;

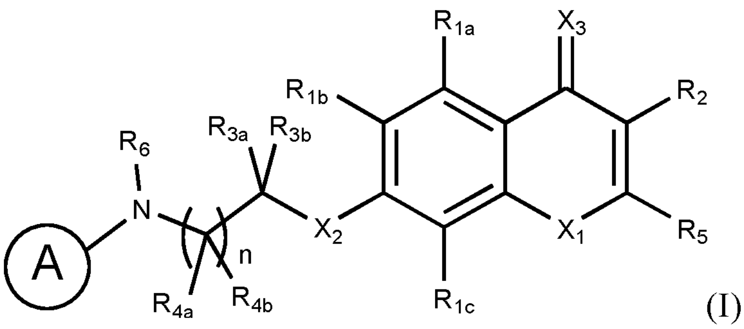
(I)

$X_1$, $X_2$ are each independently selected from the group consisting of: O, $NR_7$; wherein said $R_7$ is selected from the group consisting of: a hydrogen, alkyl, substituted alkyl, arylalkyl, substituted arylalkyl, cycloalkyl, substituted cycloalkyl;
$X_3$ is selected from the following group: O, $NR_8$; wherein said $R_8$ is selected from the following group: a hydrogen, alkyl;
$R_{1a}$, $R_{1b}$, $R_{1c}$ are each independently selected from the following group: a hydrogen, hydroxyl, alkoxy;
$R_2$ is selected from the following group: a substituted or unsubstituted aryl, or substituted or unsubstituted heteroaryl;
$R_{3a}$, $R_{3b}$ are each independently selected from the following group: a hydrogen, alkyl, halogen;
$R_{4a}$, $R_{4b}$ are each independently selected from the following group: a hydrogen, alkyl, halogen;
$R_5$ is selected from the following group: a hydrogen or alkoxy;
$R_6$ is selected from the following group: a hydrogen, substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, substituted or unsubstituted alkoxycarbonyl, substituted or unsubstituted carbamoyl, or $(CH_2)_tR_7$; wherein t is 1, 2, 3, 4, 5 or 6; wherein said $R_7$ is selected from the following group: a $C_2$-$C_6$ acyl, $C_2$-$C_6$ amido,

aryl, heteroaryl, cycloalkyl, heterocycloalkyl, $NR_8COR_9$; Said $R_8$ is selected from the following group: a hydrogen, alkyl; $R_9$ is selected from the following group: an alkyl, aryl, heteroaryl, cycloalkyl, heterocycloalkyl;

The A ring is a substituted or unsubstituted cycloalkyl;
n is 1, 2, 3, 4, 5, 6, 7, 8, 9 or 10;
Wherein each ""substituted" means that a hydrogen atom on a group is replaced by one or more substituents selected from the group consisting of: a cyano, halogen, alkyl, hydroxy, alkoxy, alkenyl, alkynyl, aryl, heteroaryl;
And the pharmaceutical composition is used for purposes selected from the following group:

(1) ameliorating, mitigating, or reversing a disease or condition caused by a viral infection;
(2) Acting as a SARS-Cov-2 viral ACE-2 receptor antagonist;
(3) As an inhibitor of post-infectious inflammatory cytokines.

In another preferred example, said virus is selected from the following group: influenza virus, respiratory syncytial virus, coronavirus (e.g., SARS-COV-2 virus), parainfluenza virus.
In another preferred example, said infection is a viral infection.
In another preferred example, said virus is selected from the following group: influenza virus, respiratory syncytial virus, coronavirus (e.g., SARS-COV-2 virus), parainfluenza virus.
It should be understood that, within the scope of the present invention, each of the above-described technical features of the present invention and each of the technical features specifically described below (e.g., in the embodiments) can be combined with each other, thereby constituting a new or preferred technical solution. For the sake of limitation of space, we will not repeat all of them herein.

## Description of drawings

Figure 1 shows a line graph of the results of the efficacy assay of compound 4 against SARS-Cov-2.
Figure 2 shows a cell membrane chromatogram of the compounds of the present invention.
Figure 3 shows a graph of the calcium imaging results of compound 4 in ACE2-293T cells.
Figure 4 shows a histogram of the effect of compound 4 on INF-$\beta$ content in lung tissue in an influenza virus FM1-infected mouse pneumonia model.
Figure 5 shows a histogram of the effect of compound 4 on TNF-$\alpha$ content in lung tissue in an influenza virus FM1-infected mouse pneumonia model.
Figure 6 shows a histogram of the effect of compound 4 on IL-1 content in lung tissue in a human coronavirus 229E-infected mouse model of pneumonia.
Figure 7 shows a histogram of the effect of compound 4 on the IL-10 content in lung tissue in a human coronavirus 229E-infected mouse pneumonia model.

## Modes for carrying out the invention

The inventors, after extensive and intensive research, unexpectedly found that the compounds of the present invention have excellent effects in the treatment of diseases caused by a variety of viral infections, and thus conducted comparative experiments between the compounds of the present invention and a variety of known antiviral drugs. On this basis, the present invention was accomplished.

## Definition

The following are definitions of terms used in this specification. Unless otherwise noted, the initial definitions of groups or terms provided herein apply to groups or terms used in this specification alone or as part of other groups.
The term "substituted" refers to any of the substituents mentioned in the specification of the present invention, including but not limited to: a halogen, nitro, cyano, carboxy, oxo, alkyl, substituted alkyl, cycloalkyl, substituted cycloalkyl, streptenyl, substituted streptenyl, alkynyl, substituted alkynyl, aryl, substituted aryl, heteroaryl, substituted heteroaryl, heterocyclic, substituted heterocyclic, hydroxy, alkoxy, aryloxy, alkanoyloxy, aryloyloxy, amino, alkylamino, arylamino, arylalkanoylamino, heteroarylalkylamino, aminoalkylamino, alkylaminoalkylamino, alkylaminoalkylamino, dialkylaminoalkylamino, alkylamino, arylamino, arylalkylamino, di-substituted amine (wherein two substituents of amino are selected from an alkyl, aryl, or arylalkyl), alkanoyl, substituted alkanoyl, arylalkyl, heteroarylalkyl, carboxylic, alkoxycarbonyl, aryloxycarbonyl, alkylaminocarbonyl, arylaminocarbonyl, arylaminocarbonyl, carboxylic, alkoxy, aryloxycarbonyl,

alkamido carbonyl, arylaminocarbonyl, arylalkoxycarbonyl, alkylsulfonyl, arylsulfonyl, arylalkylsulfonyl, carbamoyl, substituted carbamoyl, substituted alkylcarbamoyl, amide, substituted amide, sulfonamide, substituted sulfonamide.

The term "halogen" or "halo" means fluorine, chlorine, bromine, iodine.

The term "alkyl" refers to a straight or branched unsubstituted hydrocarbon group having 1 to 20 carbon atoms, preferably 1 to 7 carbon atoms. Examples of "alkyl" include, but are not limited to, methyl, ethyl, 1-propyl, 2-propyl, 1-butyl, 2-butyl and the like.

The term "substituted alkyl" means an alkyl group substituted with 1-4 substituents, such as: a halogen, nitro, cyano, carboxy, oxo, alkyl, substituted alkyl, cycloalkyl, substituted cycloalkyl, streptenyl, substituted streptenyl, alkynyl, substituted alkynyl, aryl, substituted aryl, heteroaryl, substituted heteroaryl, heterocyclic, substituted heterocyclyl, hydroxy, alkoxy, aryloxy, alkanoyloxy, aryloyloxy, amino, alkylamino, arylamino, arylalkylamino, disubstituted amine (wherein said two substituents for amino are selected from an alkyl, aryl, or arylalkyl), alkanoyl, substituted alkanoyl, alkoxycarbonyl, arylalkoxycarbonyl, alkylsulfonyl, arylsulfonyl, arylalkylsulfonyl, carbamoyl, substituted carbamoyl, amide, substituted amide, sulfonamide, substituted sulfonamide.

The term "alkenyl" means a straight or branched hydrocarbon group having 2-20 carbon atoms, preferably 2-15 carbon atoms, most preferably 2-8 carbon atoms, and 1-4 double bonds.

The term "substituted alkenyl" means an alkenyl group substituted by 1-2 substituents such as: a halogen, nitro, cyano, aryl, substituted aryl, heteroaryl, substituted heteroaryl, alkoxy, aryloxy, alkanoyloxy, aryloxy, amino, alkylamino, arylamino, arylalkylamino, disubstituted amine (wherein two substituents for amino are selected from an alkyl, aryl or arylalkyl).

The term "alkynyl" means a straight or branched hydrocarbon group having 2-20 carbon atoms, preferably 2-15 carbon atoms, most preferably 2-8 carbon atoms, and 1-4 triple bonds.

The term "substituted alkynyl" means an alkynyl group substituted by, for example, a halogen, nitro, cyano, aryl, substituted aryl, heteroaryl, substituted heteroaryl, hydroxyl, alkoxy, aryloxy, alkanoyloxy, aryloyloxy, amino, alkylamino, arylamino, arylalkylamino, disubstituted amine (wherein two substituents for amino are selected from an alkyl, aryl or arylalkyl).

The term "aryl" means a monocyclic or bicyclic aromatic hydrocarbon group having 6-12 carbon atoms in the ring portion. Aryl groups include dicyclic groups that are fused to a saturated or partially unsaturated aromatic ring, or an aromatic carbocyclic or heterocyclic ring. Typically, aryl groups include, but are not limited to, the following groups: benzene, naphthalene, anthracene, biphenyl, 1,2-dihydronaphthalene, 1,2,3,4-tetrahydronaphthalene, and the like.

The term "substituted aryl" means an aryl group substituted with 1-4 substituents such as: a halogen, halo, nitro, cyano, ureido, carboxyl, trifluoromethoxy, trifluoromethyl, alkyl, substituted alkyl, cycloalkyl, substituted cycloalkyl, streptenyl, substituted streptenyl, alkynyl, substituted alkynyl, aryl, substituted aryl, heteroaryl, substituted heteroaryl, heterocyclic, substituted heterocyclic, hydroxy, alkoxy, aryloxy, alkanoyloxy, aryloxy, amino, alkylamino, arylamino, arylalkylamino, disubstituted amine (wherein two substituents for amino are selected from an alkyl, aryl, or arylalkyl), alkanoyl, substituted alkanoyl, alkoxycarbonyl, arylalkoxycarbonyl, alkylsulfonyl, aryl sulfonyl, aryl alkyl sulfonyl, carbamoyl, substituted carbamoyl, amide, substituted amide, sulfonamido, substituted sulfonamido.

The term "cycloalkyl" refers to a non-aromatic, saturated or partially unsaturated cyclic hydrocarbon group, said cycloalkyl group being arbitrarily substituted with one or more of the substituents described in the present application, having 3-30 carbon atoms for a monocyclic ring, or 7-12 carbon atoms for a bicyclic ring. Examples of monocyclic cycloalkyl groups include, but are not limited to, cyclopropyl, cyclobutyl, cyclopentyl, 1-cyclopenta-1-enyl, 1-cyclopenta-2-enyl, 1-cyclopenta-3-enyl, cyclohexyl, 1-cyclohex-1-enyl, cycloheptyl, cyclooctyl. Exemplary bridge-forming bicyclic cycloalkyl groups include, but are not limited to, bicyclo[2.2.1]heptane, bicyclo[2.2.2]octane, bicyclo[3.2.2]nonane.

The terms "heterocycle", "heterocyclic" and "heterocyclyl" refer to optionally substituted, fully saturated or unsaturated, aromatic or non-aromatic cyclic groups, for example, which can be a 4-7-membered monocyclic, 7-11-membered bicyclic or 10-15-membered tricyclic system having at least one heteroatom in at least one ring containing a carbon atom. Each ring of the heterocyclic group containing a heteroatom may have 1, 2 or 3 heteroatoms selected from nitrogen atoms, oxygen atoms and sulfur atoms. Said "heterocyclic group" may be arbitrarily substituted with one or more of the substituents described herein, and examples of "heterocyclic groups" include, but are not limited to, pyrrolidinyl, tetrahydrofuranyl, tetrahydropyranyl, morpholino, thiomorpholino, piperazinyl, homopiperazinyl, epoxypropyl, imidazolyl, imidazolyl, homopiperazinyl, epoxypropyl, imidazolidinyl, 3-azabicyclo[3.1.0]hexyl, 3-azabicyclo[4.1.0]heptyl, azabicyclo [2.2.2]hexyl, N-pyridinylurea, pyrimidinyl ketone, and 1,1-dioxo thiomorpholinyl.

The term "heteroaryl" refers to a monovalent aromatic group of 5-, 6-, 7-, 8-, 9-, or 10-membered-ring and comprises a 5-20 atom fused system containing one or more heteroatoms selected from nitrogen, oxygen, phosphorus, and sulfur, which may be arbitrarily substituted by one or more of the substituents described in this application. Examples of "heteroaryl" include, but are not limited to, pyridinyl, imidazolyl, imidazopyridinyl, pyrimidinyl, pyrazolyl, triazolyl, pyrazinyl, tetrazolyl, furanyl, thiophenyl, thiazolyl, quinolyl, indolyl, and the like.

The term "oxo-substituent" stands for divalent group=O.

The term "carbamoyl" refers to the $-OC(=O)NH_2$ group.

The term "amide" refers to the $-C(=O)NH_2$ group.

The term "sulfonamide group" refers to the $-SO_2NH_2$ group.

The terms "substituted carbamoyl", "substituted amide", "substituted sulfonamide" mean that the amide, sulfonamide or carbamate has at least one hydrogen substituted with a group selected from an alkyl, substituted alkyl, streptenyl, substituted streptenyl, cycloalkyl, substituted cycloalkyl, aryl, substituted aryl, heteroaryl, substituted heteroaryl, heterocyclyl, substituted heterocyclyl.

The term "acceptable salt" refers to a pharmaceutically acceptable organic or inorganic salt of a compound of the invention. Exemplary salts include, but are not limited to, sulfates, citrates, acetates, oxalates, chlorides, bromides, iodides, nitrates, acid sulfates, isonicotinic acid salts, lactates, salicylates, acid citrates, succinate, maleates, fenugreek, gluconate, formate, methane sulfonate, and pamoate. "Acceptable salt" may relate to including another molecule such as a maleate or other counterion. The counterion stabilizes the charge in the parent compound. The "acceptable salt" may have more than one charged atom, and the plurality of charged atoms may have a plurality of counterions.

If the compound of the invention is a base, the desired "acceptable salt" can be prepared by suitable methods, for example, by treating the free base with one of the following inorganic acids: hydrochloric acid, hydrobromic acid, sulfuric acid, nitric acid, phosphoric acid; or one of the following organic acids: acetic acid, maleic acid, succinic acid, mandelic acid, fumaric acid, malonic acid, pyruvic acid, salicylic acid, pyranosidic acid such as glucuronic acid or galacturonic acid, alpha hydroxy acids such as citric acid or tartaric acid, amino acids such as glutamic acid, aromatic acids such as benzoic acid or cinnamic acid, and sulphonic acids such as methanesulfonic acid or p-toluenesulfonic acid.

If the compound of the present invention is an acid, the desired "acceptable salt" can be prepared by suitable methods, e.g., by treating the free acid with an inorganic or organic base such as an amine, an alkali metal hydroxide or an alkaline earth metal hydroxide. Exemplary examples of suitable salts include, but are not limited to, organic salts derived from amino acids, primary, secondary and tertiary amine salts, and salts of cyclic amines such as piperidines, morpholines and piperazines, as well as inorganic salts derived from sodium, calcium, potassium, magnesium, manganese, iron, copper, zinc, aluminum and lithium.

A solvate is a combination or complex of one or more solvent molecules with a compound of the present invention. Examples of solvents that form solvent compounds include, but are not limited to, water, isopropanol, ethanol, methanol, dimethyl sulfoxide, ethyl acetate, acetic acid, and ethanolamine. The compounds of the present invention can exist in a non-solvated form or in a solvated form with pharmaceutically acceptable solvents such as water, ethanol, etc., so the present invention will include both solvated and non-solvated forms.

The compounds of the present invention may contain asymmetric or chiral centers and, as a result, different stereoisomeric forms exist. All stereoisomeric forms of the compounds of the present invention, including, but not limited to, diastereomeric, enantiomeric, and site-resistant isomers, and mixtures thereof, such as racemic mixtures, will form part of the present invention. All stereoisomers are considered herein when the stereochemistry of any particular chiral atom is not determined. Furthermore, the present invention relates to all geometrical and positional isomers. The compounds of the present invention may exist in different reciprocal isomeric forms and all such forms are included within the scope of the present invention. All stereoisomers of the compounds of the present invention are expected to comprise either a mixture form or a pure or substantially pure form. They can be split by physical methods such as stepwise crystallization, separation or crystallization of diastereomeric derivatives, or separation by chiral column chromatography.

The compounds of the present invention may be used alone or in combination with other therapeutic agents. Combination therapy may provide a synergistic effect, i.e. the effect achieved when the active ingredients are used together is greater than the sum of the effects produced by using said compounds separately.

Said combination treatment may be administered in a simultaneous or sequential regimen. When administered sequentially, said combination may be administered in two or more uses. Compounds may be administered together in a single pharmaceutical combination or separately, and when administered separately, may be administered simultaneously or sequentially in any order.

The compounds of the present invention may be administered by any route suitable for the condition being treated. Suitable routes include, but are not limited to, oral, extragastrointestinal (including subcutaneous, intramuscular, intravenous, intra-arterial, intradermal), vaginal, intraperitoneal, intrapulmonary, and intranasal route. It should be understood that the preferred route may vary depending on, for example, the patient’s medical condition. When said compounds are administered orally, they can be formulated into pills, capsules, tablets, etc. with pharmaceutically acceptable carriers or excipients. When said compound is formulated parenterally, it may be formulated with a pharmaceutically acceptable parenteral carrier.

The present invention allows for the administration of the compounds in any convenient formulation, and by "formulation" the present invention means a dosage form containing a compound of general formula I of the present invention which facilitates drug delivery, such as, but not limited to, aqueous injections, powders, pills, powders, powders, tablets, patches, suppositories, creams, gels, granules, capsules, aerosols, sprays, powders, extended-release and controlled-release dosages, and the like. These pharmaceutical excipients can be either routinely used in various preparations, such as, but not limited to, isotonic agents, buffers, flavor enhancers, excipients, fillers, adhesives,

disintegrating agents and lubricants, etc.; or they can be selected for use in order to be compatible with the said substances, such as emulsifiers, solubilizers, bacteriostatic agents, painkillers and antioxidants, etc., which are effective in improving the stability and solubility or change the release rate and absorption rate of the compounds, etc., thereby improving the metabolism of the compounds of the present invention in the organism and thus enhancing the effect of drug delivery. In addition, excipients such as, but not limited to, gelatin, albumin, chitosan, polyether and polyester polymer materials such as, but not limited to, polyethylene glycol, polyurethane, polycarbonate and copolymers thereof may be used to achieve a specific purpose or mode of drug delivery, e.g., sustained-release, controlled-release and pulsatile drug delivery. The main manifestations of "facilitating drug delivery" include, but are not limited to, improved therapeutic efficacy, improved bioavailability, reduced toxicity and improved patient compliance.

In the following embodiments, only some of the embodiments of the present invention are given in a manner that clarifies the method of the present invention. However, these embodiments do not limit the scope of the present invention in any way, and simple improvements to the method of preparation of the present invention within the conceptual premise of the present invention are within the scope of the claimed protection of the present invention. That is, when the present invention is described in connection with the enumerated embodiments, it should be understood that it is not intended to limit the present invention to those embodiments. Rather, the invention is intended to encompass all variations, improvements and equivalent forms. Those skilled in the art will recognize many methods and substances similar or equivalent to those described in this application, which may be used to achieve the present invention.

In the embodiments described below, all temperatures are given in degrees Celsius unless otherwise indicated. Unless otherwise noted, reagents were acquired or customized from commercial suppliers, such as National Pharmaceuticals, Shaoyuan, Anegi, TCI, Sigma, and the like.

Example 1 Synthesis of Compound 1

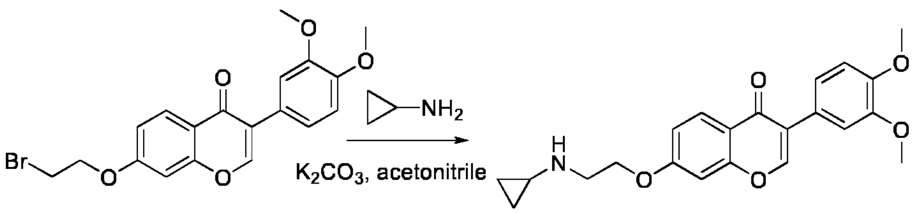


In a 50 mL round bottom flask, the substrate (0.5 mmol) was weighed and dissolved in 27 mL of acetonitrile, and organic amines (5.0 mmol), and $K_2CO_3$ (1.0 mmol) were added, heated and refluxed overnight. The end of the reaction was detected by TLC. The solvent was evaporated to dryness under a reduced pressure and 50 ml of water and 100 ml of ethyl acetate were added. The organic layer was was separated and dried over anhydrous $Na_2SO_4$ and concentrated to dryness, and the target compound was obtained in 38% yield using silica gel column chromatography.

Example 2 Synthesis of Compound 2

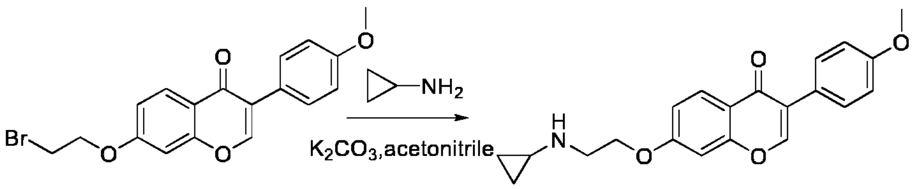


In a 50 mL round bottom flask, the substrate (0.5 mmol) was weighed and dissolved in 27 mL of acetonitrile, and organic amines (5.0 mmol), and $K_2CO_3$ (1.0 mmol) were added, heated and refluxed overnight. The end of the reaction was detected by TLC. The solvent was evaporated to dryness under a reduced pressure and 50 ml of water and 100 ml of ethyl acetate were added. The organic layer was separated and dried over anhydrous $Na_2SO_4$ and concentrated to dryness, and the target compound was obtained in 44% yield using silica gel column chromatography.

$^1H$ NMR (500 MHz, $CDCl_3$) δ 8.23 (d, *J* = 8.9 Hz, 1H), 7.97 (s, 1H), 7.58 (dt, *J* = 3.1, 1.8 Hz, 2H), 7.53 - 7.34 (m, 3H), 6.98 (dt, *J* = 28.2, 14.1 Hz, 1H), 6.90 (d, *J* = 2.3 Hz, 1H), 4.47 - 4.06 (m, 2H), 4.03 - 3.59 (m, 2H), 2.86 - 2.72 (m, 1H), 2.66 - 2.47 (m, 2H), 1.18 (t, *J* = 7.4 Hz, 3H), 1.04 - 0.74 (m, 4H).

$^{13}C$ NMR (126 MHz, DMSO) δ 174.65, 163.09, 159.02, 157.43, 153.50, 130.10, 126.95, 124.09, 123.36, 117.55, 115.10, 113.63, 101.04, 68.31, 55.17, 47.62, 40.02, 39.85, 39.69, 39.52, 39.35, 39.19, 39.02, 30.04, 6.20.

**Example 3 Synthesis of Compound 3**

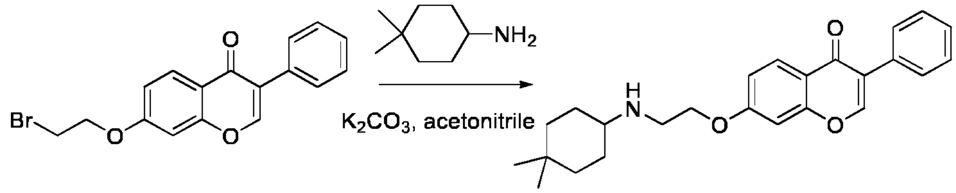


In a 50 mL round bottom flask, the substrate (0.5 mmol) was weighed and dissolved in 27 mL of acetonitrile, and organic amines (5.0 mmol), and $K_2CO_3$ (1.0 mmol) were added, heated and refluxed overnight. The end of the reaction was detected by TLC. The solvent was evaporated to dryness under a reduced pressure and 50 ml of water and 100 ml of ethyl acetate were added. The organic layer was separated and dried over anhydrous $Na_2SO_4$ and concentrated to dryness, and the target compound was obtained in 42% yield using silica gel column chromatography.

**Example 4 Synthesis of Compound 4**

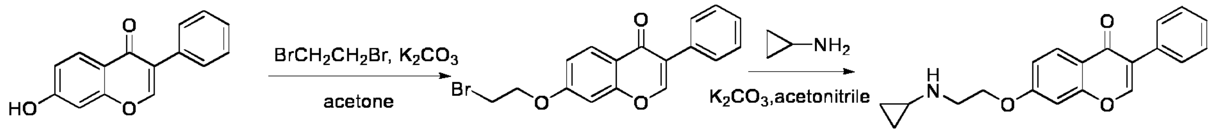


Step 1: Compound 7-hydroxyisoflavone (0.040 mol), anhydrous acetone 400 mL, 1,2-dibromoethane (0.403 mol) and potassium carbonate (0.080 mol) were added to a 1 L round-bottomed flask and heated to reflux overnight. The end of the reaction was detected by TLC, the solvent was evaporated to dryness under a reduced pressure, and 10 mL of water and 300 mL of ethyl acetate was added. The organic layer was separated and dried over anhydrous $Na_2SO_4$ and concentrated to dryness, and 7-(2-bromoethoxy)isoflavone (12.98 g) was obtained in 94% yield as a white solid using silica gel column chromatography.

Step 2: Compound 7-(2-bromoethoxy)isoflavone (0.5 mmol), $CH_3CN$ 27 mL and cyclopropylamine (1.0 mmol) were sequentially added to a 50 mL round-bottomed flask, and then $K_2CO_3$ (1.0 mmol) was added and heated to reflux overnight. The end of the reaction was detected TLC. The solvent was evaporated to dryness under a reduced pressure and 50 ml of water and 100 ml of ethyl acetate were added. The organic layer was separated and dried over anhydrous $Na_2SO_4$ and concentrated to dryness, and the target compound was obtained in 53% yield using silica gel column chromatography.

$^1$HNMR (400 MHz, DMSO) δ 8.46 (s,1H), 8.04 (d, *J* = 8.8 Hz, 1H), 7.64 -7.35 (m, 5H), 7.16 (s, 1H), 7.09 (d, *J* =8.8 Hz, 1H), 4.17 (t, *J* = 5.4 Hz, 2H),2.99 (t, *J* = 5.3 Hz, 2H), 2.23 - 2.09 (m,1H), 0.46 - 0.18 (m, 4H).

$^{13}$CNMR (101 MHz, DMSO) δ 174.4, 163.1, 157.4, 154.1 , 132.0, 128.9, 128.1 ,127.8, 127.0, 123.8, 117.6, 115.1 , 101.1 ,68.3, 47.6, 30.0, 6.2.

HRMS (ESI): m/z, calcd for $[M+H]^+$:322.1438; found: 322.1439.

**Example 5 Synthesis of Compound 5**

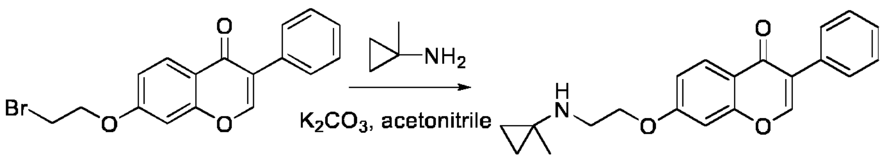

Compound 7-(2-bromoethoxy)isoflavone (0.5 mmol), $CH_3CN$ 27 mL and 1-methyl-cyclopropylamine (1.0 mmol) were sequentially added to a 50 mL round-bottomed flask, and then $K_2CO_3$ (1.0 mmol) was added, and heated to reflux overnight. The end of the reaction was detected by TLC. The solvent was evaporated to dryness under a reduced pressure and 50 ml of water and 100 ml of ethyl acetate were added. The organic layer was separated and dried over anhydrous $Na_2SO_4$ and concentrated to dryness, and the target compound was obtained in 37% yield using silica gel column chromatography.

$^1H$ NMR (500 MHz, $CDCl_3$) δ 8.20 (d, *J* = 8.9 Hz, 1H), 7.93 (s, 1H), 7.63 - 7.49 (m, 2H), 7.47 - 7.40 (m, 2H), 7.40 - 7.33 (m, 1H), 6.99 (dd, *J* = 8.9, 2.4 Hz, 1H), 6.85 (d, *J* = 2.3 Hz, 1H), 4.14 (t, *J* = 5.3 Hz, 2H), 3.13 (t, *J* = 5.3 Hz, 2H), 1.32 (s, 3H), 0.71 - 0.57 (m, 2H), 0.48 - 0.36 (m, 2H).

$^{13}C$ NMR (126 MHz, $CDCl_3$) δ 175.73, 163.36, 157.97, 152.80, 132.06, 129.09, 128.58, 128.22, 127.91, 125.38, 118.61, 115.00, 100.81, 68.75, 44.82), 34.98, 22.10, 14.60.

HRMS(ESI): m/z $[M+H]^+$ calcd for$C_{21}H_{22}NO_3^+$: 326.1600; found: 336.1529.

**Example 6 Synthesis of Compound 6**

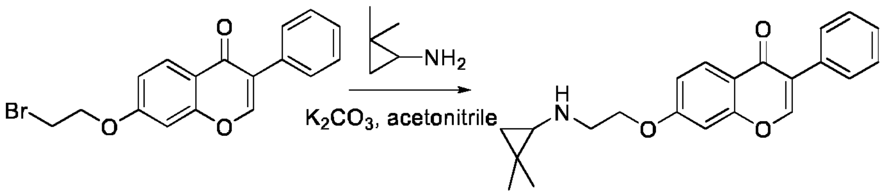


In a 50 mL round bottom flask, the substrate (0.5 mmol) was weighed and dissolved in 27 mL of acetonitrile, and organic amines (5.0 mmol) and $K_2CO_3$ (1.0 mmol) were added, heated and refluxed overnight. The end of the reaction was detected by TLC. The solvent was evaporated to dryness under a reduced pressure and 50 ml of water and 100 ml of ethyl acetate were added. The organic layer was separated and dried over anhydrous $Na_2SO_4$ and concentrated to dryness, and the target compound was obtained in 32% yield using silica gel column chromatography.

**Example 7 Synthesis of Compound 7**

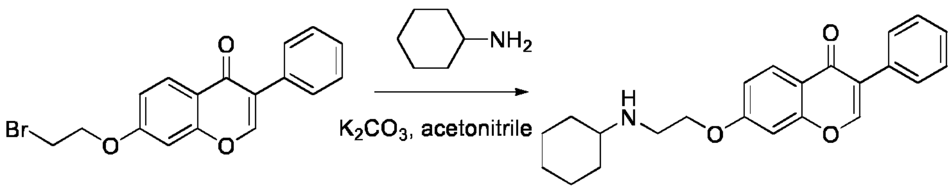


Compound 7-(2-bromoethoxy)isoflavone (0.5 mmol), $CH_3CN$ 27 mL and 1-methyl-cyclopropylamine (1.0 mmol) were sequentially added to a 50 mL round-bottomed flask, and then $K_2CO_3$ (1.0 mmol) was added and heated to reflux overnight. The end of the reaction was detected by TLC. The solvent was evaporated to dryness under a reduced pressure and 50 ml of water and 100 ml of ethyl acetate were added. The organic layer was separated and dried over anhydrous $Na_2SO_4$ and concentrated to dryness, and the target compound was obtained in 45% yield using silica gel column chromatography.

$^1H$ NMR (400 MHz, DMSO) δ 8.46 (s,1H), 8.03 (d, *J* = 8.9 Hz, 1H), 7.72 -7.30 (m, 5H), 7.17 (s, 1H), 7.09 (d, *J* =8.9 Hz, 1H), 4.16 (t, *J* = 5.4 Hz, 2H),2.95 (t, *J* = 5.4 Hz, 2H), 2.43 (t, *J* = 10.0Hz, 1H), 2.33 (d, *J* = 65.9 Hz, 1H), 1.88- 1.49 (m, 5H), 1.26 - 0.98 (m, 5H).

$^{13}C$ NMR (101 MHz, DMSO) δ 174.4,163.1, 157.4, 154.2, 132.0, 128.9, 128.1 ,127.8, 127.0, 123.8, 117.6, 115.2, 101.1 ,68.6, 56.0, 44.7, 32.6, 25.8, 24.4.

HRMS(ESI): m/z $[M+H]^+$ calcd for$C_{23}H_{26}NO_3^+$: 364.1907; found:364.1909.

**Example 8 Synthesis of Compound 8**

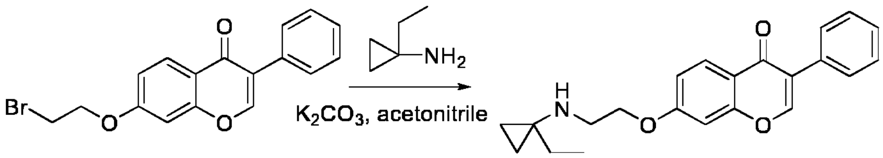


In a 50 mL round bottom flask, the substrate (0.5 mmol) was weighed and dissolved in 27 mL of acetonitrile, and organic amines (5.0 mmol), and $K_2CO_3$ (1.0 mmol) were added, heated and refluxed overnight. The end of the reaction was detected by TLC. The solvent was evaporated to dryness under a reduced pressure and 50 ml of water and 100 ml of ethyl acetate were added. The organic layer was separated and dried over anhydrous $Na_2SO_4$ and concentrated to dryness, and the target compound was obtained in 32% yield using silica gel column chromatography.

**Example 9 Synthesis of Compound 9**

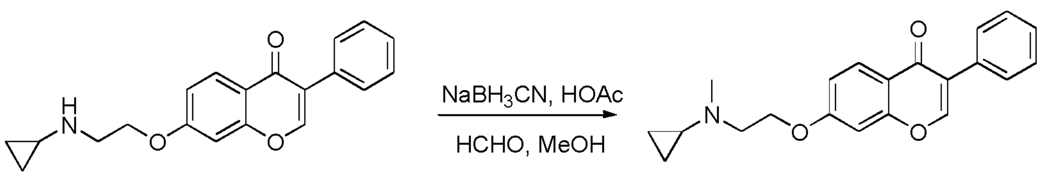


Compound 4 (2 mmol) was added sequentially in a 50 mL round bottom flask and dissolved in 20 ml of methanol. Then paraformaldehyde (20 mmol), sodium cyanoborohydride (10 mmol), and acetic acid (4 mmol) were added and stirred overnight at room temperature. The end of the reaction was detected by TLC. The solvent was evaporated to dryness under a reduced pressure, 100 ml of saturated sodium carbonate solution and 200 mL of ethyl acetate were added. The organic layer was separated, washed with saturated sodium chlorid dried over anhydrous $Na_2SO_4$ and concentrated to dryness. The target compound was obtained by silica gel column chromatography in 72% yield.

$^1H$ NMR (500 MHz, $CDCl_3$) δ 8.22 (d, *J* = 5.7 Hz, 1H), 7.86 (d, *J* = 91.6 Hz, 1H), 7.43 (dd, *J* = 82.0, 59.1 Hz, 5H), 7.03 (s, 1H), 6.88 (s, 1H), 4.22 (s, 2H), 3.04 (s, 2H), 2.50 (s, 3H), 1.78 (s, 1H), 0.51 (d, *J*= 16.3 Hz, 4H).

$^{13}C$ NMR (126 MHz, $CDCl_3$) δ 175.63, 163.31, 157.91, 152.67, 132.02, 129.01, 128.49, 128.13, 127.77, 125.26, 118.46, 115.03, 100.76, 66.55, 56.67, 43.70, 38.66, 6.91..

HRMS(ESI): m/z $[M+H]^+$ calcd for$C_{21}H_{22}NO_3^+$: 336.16; found: 336.1672.

**Example 10 Synthesis of Compound 10**

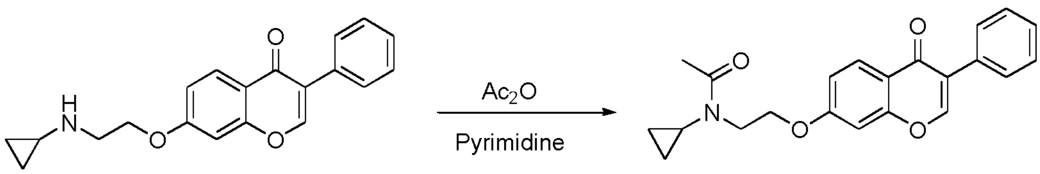


Compound 4 (2 mmol) was added sequentially in a 50 mL round bottom flask and dissolved in 20 ml of pyridine. Then acetic anhydride (20 mmol) was added and stirred overnight at room temperature. The end of the reaction was detected by TLC. The solvent was evaporated to dryness under a reduced pressure, and 100 ml of 1M hydrochloric acid and 200 mL of ethyl acetate were added. The organic layer was separated, and washed with 1M hydrochloric acid, saturated sodium bicarbonate and saturated sodium chloride , respectively. The organic layer was dried over anhydrous $Na_2SO_4$ and concentrated to dryness, and the target compound was obtained by silica gel column chromatography in 81% yield.

$^1H$ NMR (500 MHz, $CDCl_3$) δ 8.25 (t, *J* = 29.2 Hz, 1H), 7.96 (s, 1H), 7.55 (t, *J* = 18.2 Hz, 2H), 7.45 (t, *J* = 7.4 Hz, 2H), 7.39 (t, *J* = 7.3 Hz, 1H), 6.98 (dt, *J* = 21.9, 10.9 Hz, 1H), 6.90 (d, *J* = 1.9 Hz, 1H), 4.47 - 4.11 (m, 2H), 3.78 (dt, *J* = 30.5, 5.4 Hz, 2H), 2.99 - 2.63 (m, 1H), 2.35 - 1.98 (m, 3H), 1.09 - 0.68 (m, 4H).

$^{13}C$ NMR (126 MHz, $CDCl_3$) δ 175.63, 174.16, 163.08, 157.95, 152.76, 131.98, 129.02, 128.51, 128.16, 127.87, 125.29, 118.59, 114.91, 100.72, 66.40, 46.77, 32.17, 22.82, 9.79.

**Example 11 Synthesis of Compound 11**

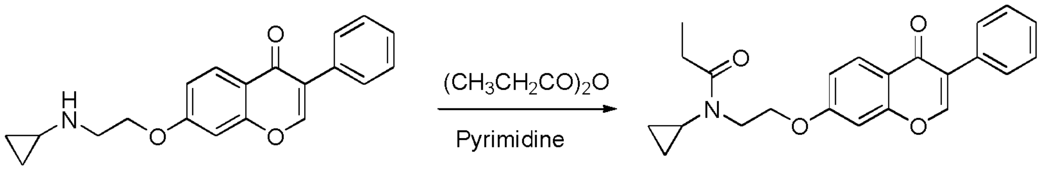


Compound 4 (2 mmol) was added sequentially in a 50 mL round bottom flask and dissolved in 20 ml of pyridine. Then propionic anhydride (20 mmol) was added and stirred overnight at room temperature. The end of the reaction was detected by TLC. The solvent was evaporated to dryness under a reduced pressure, 100 ml of 1M hydrochloric acid and 200 mL of ethyl acetate were added. The organic layer was separated, and washed with 1M hydrochloric acid, saturated sodium bicarbonate and saturated sodium chloride , respectively. The organic layer was dried over anhydrous $Na_2SO_4$ and concentrated to dryness, and the target compound was obtained by silica gel column chromatography in 83% yield.

$^1H$ NMR (500 MHz, $CDCl_3$) δ 8.23 (d, *J*= 8.9 Hz, 1H), 7.97 (s, 1H), 7.58 (dt, *J* = 3.1, 1.8 Hz, 2H), 7.53 - 7.34 (m, 3H), 6.98 (dt, *J* = 28.2, 14.1 Hz, 1H), 6.90 (d, *J* = 2.3 Hz, 1H), 4.47 - 4.06 (m, 2H), 4.03 - 3.59 (m, 2H), 2.86 - 2.72 (m, 1H), 2.66 - 2.47 (m, 2H), 1.18 (t, *J* = 7.4 Hz, 3H), 1.04 - 0.74 (m, 4H).

$^{13}C$ NMR (126 MHz, $CDCl_3$) δ 177.35, 175.70, 163.17, 158.02, 152.79, 132.03, 129.07, 128.56, 128.21, 127.93, 125.36, 118.62, 114.97, 100.75, 66.53, 47.02, 31.41, 27.56, 9.73, 9.23.

**Example 12 Synthesis of Compound 12**

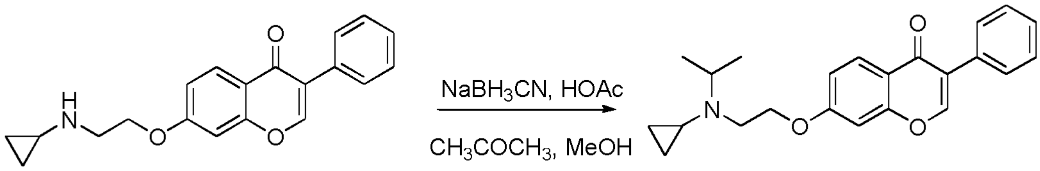


Compound 4 (2 mmol) was added sequentially in a 50 mL round bottom flask and dissolved in 20 ml of methanol. Then acetone (20 mmol), sodium cyanoborohydride (10 mmol), and acetic acid (4 mmol) were added and stirred overnight at room temperature. The end of the reaction was detected by TLC. The solvent was evaporated to dryness under a reduced pressure, 100 ml of saturated sodium carbonate solution and 200 mL of ethyl acetate were added. The organic layer was separated, and washed with saturated sodium chloride. The organic layer was dried over anhydrous $Na_2SO_4$ and concentrated to dryness, and the target compound was obtained by silica gel column chromatography in 75% yield.

1H NMR (500 MHz, $CDCl_3$) δ 8.21 (d, *J* = 8.9 Hz, 1H), 7.95 (s, 1H), 7.60 - 7.52 (m, 2H), 7.44 (dd, *J* = 10.2, 4.7 Hz, 2H), 7.41 - 7.35 (m, 1H), 6.99 (dd, *J* = 8.9, 2.3 Hz, 1H), 6.86 (d, *J* = 1.6 Hz, 1H), 4.15 (s, 2H), 3.14 (s, 1H), 3.03 (s, 2H), 1.98 (s, 1H), 1.10 (s, 6H), 0.49 (d, *J* = 37.0 Hz, 4H).

$^1H$ NMR (500 MHz, MeOD) δ 8.25 (s, 1H), 8.13 (d, *J* = 9.3 Hz, 1H), 7.58 - 7.52 (m, 2H), 7.44 (t, *J* = 7.4 Hz, 2H), 7.41 - 7.36 (m, 1H), 7.11 - 7.06 (m, 2H), 4.25 (t, *J* = 6.1 Hz, 2H), 3.22 - 3.13 (m, 1H), 3.07 (t, *J* = 6.1 Hz, 2H), 2.11 - 1.92 (m, 1H), 1.14 (d, *J* = 6.7 Hz, 6H), 0.59 - 0.52 (m, 2H), 0.49 - 0.43 (m, 2H).

$^{13}C$ NMR (126 MHz, $CDCl_3$) δ 175.71, 163.51, 158.03, 152.69, 132.10, 129.08, 128.55, 128.17, 127.84, 125.33, 118.44, 115.09, 100.76, 68.10, 53.25, 49.13, 34.54, 18.97, 7.23.

HRMS(ESI): m/z $[M+H]^+$ calcd for$C_{23}H_{26}NO_3^+$: 364.1913; found: 364.1906.

The chemical structures that appear in the table or figure below are shown below:

| | |
|---|---|
| 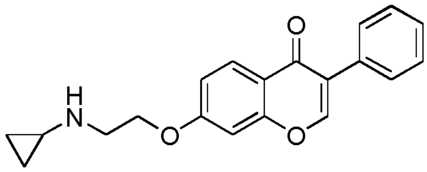 Compound 4 | 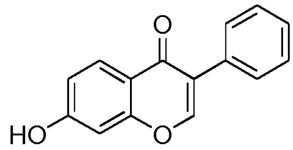 Compound F1 |

| | |
|---|---|
| 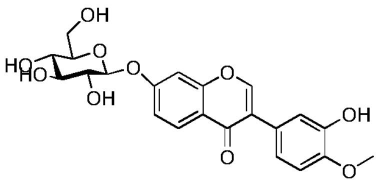 Compound F2 | 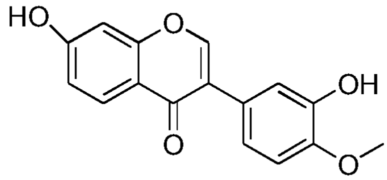 Compound F3 |
| 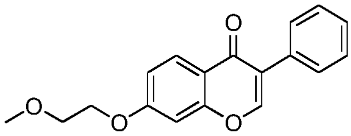 Compound F4 | 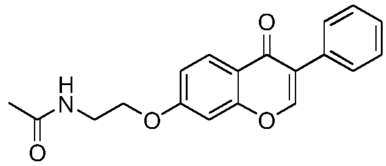 Compound F5 |
| 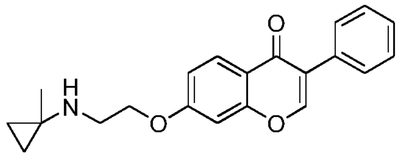 Compound 5 | 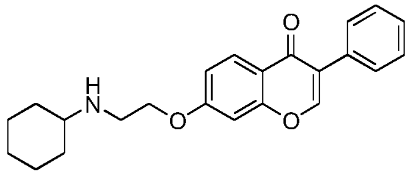 Compound 7 |
| 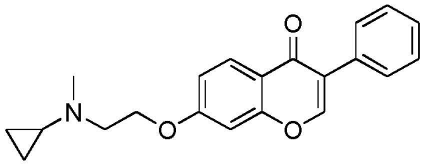 Compound 9 | 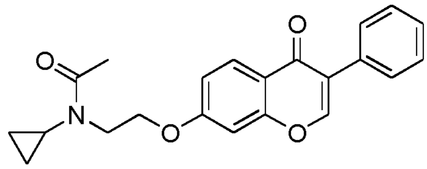 Compound 10 |
| 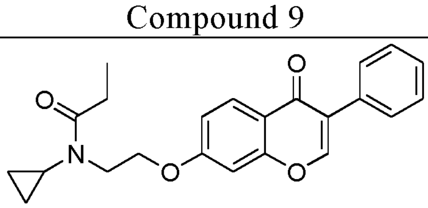 Compound 11 | 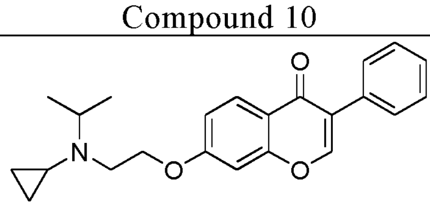 Compound 12 |

**Example 13: Therapeutic effects of compounds of the present invention on** a **mouse model of pneumonia infected with influenza A (H1N1) virus strain FM1**

**1 Experimental materials**

**1.1** Tested drugs: Compound 4, Compound F1, Compound F2, Compound F3

**1.2** Positive control drugs

Ribavirin Granules: Manufactured by Sichuan Baili Pharmaceutical Co. Indications: This product is suitable for viral pneumonia, bronchitis, and skin herpes virus infection caused by respiratory syncytial virus. Main components: ribavirin. Properties: White or off-white soluble granules. Specification: 50 mg/bag, 18 bags/box. Usage and dosage: Oral administration, 150 mg once, 3 times a day. Storage conditions: airtight, stored in a dry place.

**1.3** Test animals

| strain | level | weight (g) | Numbe r (a) | gender | Certificate of Conformity number |
|---|---|---|---|---|---|
| ICR mouse | SPF grade | 13~15 | 110 | 50/50 male and female | 11400700372636 |
| Animal source | Beijing Viton Lihua Laboratory Animal Technology Co. | | | | |
| license number | SCXK(jing)2016-0006 | | | | |

**1.4** Virus strains

| Virus strain name | source | use |
|---|---|---|
| Influenza A (H1N1) virus FM1 strain | ATCC | Construction of an influenza virus-infected mouse pneumonia model |

**1.5** Test instruments

| Instrument Name | model number | manufacturer | Instrument Usage |
|---|---|---|---|
| A2 type biological safety cabinet | Thermo MSC1.8 | Thermo Corporation, USA | Animal infections, anatomy |
| Electronic balance | YP1002 MAX100g | Shanghai Yue Ping Scientific Instrument Co. | Weighing of mice |
| Electronic balance | AL204 MAX210g | METTLER TOLEDO INSTRUMENTS | Weighing tissue weight |
| IVC Mouse Cages | ZJ-4 | Suzhou Fung Co. | Mouse feeding |

**2 Test Methods**

**2.1** Dosage design and formulation

Drug on test

| | |
|---|---|
| Mouse dosage | Two doses, 50mg/kg and 25mg/kg were used in the experiment. |
| Formulation concentration | The solutions were prepared into corresponding concentrations with CMC-Na respectively, and the mice were administered by gavage at 0.2 ml/10 g/d once a day during the test. |

Ribavirin granules

| | |
|---|---|
| Human clinical dose | 450mg/60kg/d |
| Mouse dose | Clinical doses were converted to mouse doses:450mg/60kg/d×11=82.5mg/kg/d |
| Dose given to mice | 20ml/kg/d, gavage |
| formulated concentration | 82.5mg/kg/d÷20ml/kg/d=4.125mg/ml |
| Formulation method | 3bags (150mg) $\xrightarrow{\text{Dissolve in distilled water until}}$ 36.36ml， 4°C storage. |

**2.2** Modeling and drug administration

ICR mice were taken and randomly divided into: normal control group, model control group, ribavirin control group, compound 4, compound F1, compound F2, and compound F3 according to their weight class, and each drug was given in two dosage groups (10 mice in each group). Except for the normal control group, mice were slightly anesthetized with ether and infected with 15 $LD_{50}$ H1N1 influenza virus liquid FM1 strain by nasal drops (30 μl per animal). The administration was started on the day of infection at 0.2 ml/10 g by gavage each time once a day for 4 days, and the normal control and model control groups were gavaged with distilled water under the same conditions. The mice were weighed and dissected on Day 5, and the lung was weighed to calculate lung index and lung index inhibition. The results were statistically processed using t-test for comparison between groups.

$$\text{Lung index (\%)} = \text{wet lung weight (g)} \times 100/\text{ body weight (g)}$$

$$\text{Lung index inhibition rate (\%)} = \frac{\text{Lung weight in model control group (g)- Lung weight of administered group (g)}}{\text{Lung weight in model control group (g)- Normal control lung weight (g)}} \times 100\%$$

**3 Test results**

**Table 1 Therapeutic effects of compounds of the present invention on a mouse model of pneumonia infected with influenza virus strain H1N1/ FM1**

| groups | dosage (mg/kg | number of animals (n) | lung index | inhibition rate (%) |
|---|---|---|---|---|
| Normal control group | - | 10 | 0.70±0.11 | -- |
| Model Control Group | - | 10 | 1.22±0.23## | -- |
| Ribavirin control group | 82.5 | 10 | 0.96±0.16** | 49.59 |
| Compound 4 | 50 | 10 | 1.01±0.12** | 39.27 |
| | 25 | 10 | 1.09±0.13 | 23.73 |
| Compound F1 | 50 | 10 | 1.06±0.13 | 29.97 |
| | 25 | 10 | 1.17±0.09 | 11.13 |
| Compound F2 | 50 | 10 | 1.12±0.17 | 19.06 |
| | 25 | 10 | 1.13±0.18 | 17.13 |
| Compound F3 | 50 | 10 | 1.31±0.22 | -18.73 |
| | 25 | 10 | 1.19±0.31 | 4.071 |
| Note: Comparison with normal group ## P<0.01; Comparison with model control group, *P<0.05 | | | | |

The results in Table 1 show that the lung index of mice infected with influenza A (H1N1) virus FM1 strain virus was significantly increased, which was significantly different from that of the normal control group (P<0.01). After 4 days of treatment with Compound 4, Compound F1, Compound F2, and Compound F3 starting on the day of infection, the 50 mg/kg/d dose group of Compound 4 significantly reduced the lung index of mice after infection, with a significant difference compared with the model control group (P<0.05). The 50mg/kg of compound F1 had a tendency to reduce the lung index; compound F2 and compound F3 had no significant effect on the lung index of mice. Compound 4 was more potent than compound F1, indicating that the 7-position substituent has an important influence on the anti-influenza viral potency of these isoflavone derivatives. Compound 4 was more potent than compounds F2 and F3, indicating that the position and structure of the substituent group have an important influence on the anti-influenza viral efficacy of these isoflavone derivatives.

**Example 14: Therapeutic effects of the compounds of the present invention on a mouse model of pneumonia infected with influenza A (H1N1) virus strains FM1 and PR8**

**1 Experimental materials**

**1.1** Tested drugs: Compound 4, Compound F4, Compound F5, Compound 5, Compound 7

**1.2** Positive control drugs

Oseltamivir phosphate capsule (Tamiflu) Manufactured by: Hoffmann · Roche Ltd, Basel, Switzerland. Indications: For the treatment of influenza A and B in adults and children aged 1 year and over; for the prophylaxis of influenza A and B in adults and adolescents aged 13 years and over. Main ingredient: Oseltamivir phosphate. Properties: The product is gray and light yellow capsule, the content is white to yellowish white powder, which may contain lumps. Specification: 75mg/capsule as oseltamivir, 10 capsules/box. Usage and dosage: Oral administration, adults and adolescents aged 13 years and above, 75mg each time, twice a day for 5 days. Storage condition: Keep sealed.

**1.3** Test animals

| strain | level | weight (g) | Numbe r (n) | gender | Certificate of Conformity number |
|---|---|---|---|---|---|
| ICR mouse | SPF grade | 13~15 | 130 | 50/50 male and female | 11001120110603 5 334/416 |
| ICR mouse | SPF grade | 13~15 | 50 | 50/50 male and | 110011191108281 9 |

| | | | | | |
|---|---|---|---|---|---|
| | | | | female | |
| license number | SCXK(jing)2016-0006 | | | | |
| Animal source | Beijing Viton Lihua Laboratory Animal Technology Co. | | | | |

**1.4** Virus strains

| Virus strain name | Source | Use |
|---|---|---|
| Influenza A (H1N1) virus FM1 strain, PR8 strain | ATCC | Construction of a model of pneumonia in mice infected with H1N1 influenza virus |

**1.5** Test instruments

| Instrument Name | Model number | Manufacturer | Instrument Usage |
|---|---|---|---|
| A2 type biological safety cabinet | Thermo MSC1.8 | Thermo Corporation, USA | Animal infections, anatomy |
| Electronic balance | YP1002 MAX100g | Shanghai Yue Ping Scientific Instrument Co. | Weighing of mice |
| Electronic balance | AL204 MAX210g | METTLER TOLEDO INSTRUMENTS | Weighing tissue weight |
| IVC Mouse Cages | ZJ-4 | Suzhou Fung Co. | Mouse feeding |

**2 Test Methods**

**2.1** Dosage design and formulation

Drug on test

| | |
|---|---|
| Mouse dosage | 40mg/kg/d and 20mg/kg/d for FM1 strain; 15mg/kg/d and 7.5mg/kg/d for PR8 strain. |
| Mouse dosa | 20ml/kg/d, gavage |
| Formulation concentration | The solutions were prepared into corresponding concentrations with CMC-Na respectively, and the mice were administered by gavage at 0.2 ml/10 g/d once a day during the test. |

Oseltamivir phosphate capsule

| | |
|---|---|
| Human clinical dose | 150mg/60kg/d |
| Mouse dose | Clinical doses were converted to mouse doses:<br>150mg/60kg/d×11=27.5mg/kg/d |
| Mouse dosa | 20ml/kg/d, gavage |

(continued)

| | |
|---|---|
| formulated concentration | 27.5mg/kg/d÷20ml/kg/d=1.375mg/ml |
| Formulation method | 1 grain (75mg) Dissolve in distilled water until 55ml, 4°C storage。 |

2.2 test procedure

ICR mice were taken and randomly divided by weight class: normal control group, model control group, oseltamivir control group, compound 4, compound F4, compound F5, compound 5, and compound 7 according to their weight class, and each drug was given in two dosage groups (10 mice in each group). Except for the normal control group, mice were slightly anesthetized with ether and infected with 15 $LD_{50}$ H1N1 influenza virus liquid (FM1/PR8 strain) by nasal drops (30 μl per each animal). The administration was started on the day of infection at 0.2 ml/10 g by gavage each time once a day for 4 days, and the normal control and model control groups were gavaged with distilled water under the same conditions. The mice were weighed and dissected on Day 5, and lung was weighed to calculate lung index and lung index inhibition. The results were statistically processed using t-test for comparison between groups.

$$\text{Lung index (\%)} = \text{wet lung weight (g)} \times 100/\ \text{body weight (g)}$$

$$\text{Lung index inhibition rate (\%)} = \frac{\text{Lung weight in model control group (g)- Lung weight of administered group (g)}}{\text{Lung weight in model control group (g)- Normal control lung weight (g)}} \times 100\%$$

**3 Test results**

**Table 2 Therapeutic effects of compounds of the present invention on a mouse model of pneumonia infected with influenza virus strain H1N1/ FM1**

| groups | dosage (mg/kg) | number of animals (a) | lung index | inhibition rate (%) |
|---|---|---|---|---|
| Normal control group | - | 10 | 0.71±0.03 | |
| Model Control Group | - | 10 | 1.10±0.21## | |
| Oseltamivir control Group | 27.5 | 10 | 0.86±0.21** | 62.07 |
| Compound 4 | 40 | 10 | 0.89±0.20** | 54.04 |
| | 20 | 10 | 1.04±0.12 | 15.07 |
| Compound F4 | 40 | 10 | 1.22±0.13 | -29.34 |
| | 20 | 10 | 1.14±0.12 | -9.25 |
| Compound F5 | 40 | 10 | 1.10±0.24 | 0.35 |
| | 20 | 10 | 1.22±0.11 | -31.31 |
| Compound 5 | 40 | 10 | 1.07±0.21 | 8.44 |
| | 20 | 10 | 1.20±0.16 | -25.08 |
| Compound 7 | 40 | 10 | 0.98±0.18 | 30.20 |
| | 20 | 10 | 1.02±0.12 | 21.02 |
| Note: Comparison with normal group ## P<0.01; Comparison with model control group, **P<0.01, *P<0.05 | | | | |

The results in Table 2 show that the lung index of mice infected with influenza A (H1N1) virus FM1 strain virus was significantly increased, which was significantly different from that of the normal control group (P<0.01). After 4 days of treatment with compounds of the present invention starting on the day of infection, the 40 mg/kg/d dose group of Compound 4 significantly reduced the lung index of mice after infection, with a significant difference compared with the

model control group (P<0.01), and lung index inhibition rate of 54.04%, and the compound 7 at a dose of 40 mg/kg/d had a tendency to reduce lung index. Compared with Compound 4, all of Compound F4, Compound F5, Compound 5, and Compound 7 decreased in potency. Among them, Compound F4 and Compound F5 are metabolites of Compound 4 in organisms, Compound 5 is a 7-position 2-(1-methylcyclopropylamino) ethoxy-substituted derivative, and Compound 7 is a 7-position 2-(cyclohexylamino) ethoxy-substituted derivative, which proves that the 7-position 2-(cyclohexylamino) ethoxy-substituted group has a very important role in influencing the potency of such isoflavone derivatives.

**Table 3 Therapeutic effects of compound 4 of the present invention on a mouse model of pneumonia infected with influenza virus strain H1N1/ PR8**

| groups | dosage (mg/kg ) | number of animals (n) | lung index | inhibition rate (%) |
|---|---|---|---|---|
| Normal control group | - | 10 | $0.67\pm0.06$ | - |
| Model Control Group | - | 10 | $1.08\pm0.13^{\#\#}$ | - |
| Ribavirin control group | 27.5 | 10 | $0.84\pm0.10^{**}$ | 58.89 |
| Compound 4 | 15 | 10 | $0.83\pm0.10^{**}$ | 60.27 |
| | 7.5 | 10 | $0.98\pm0.11$ | 23.91 |

Note: Comparison with normal group $^{\#\#}$ P<0.01; Comparison with model control group, *P<0.05, **P<0.01

The results in Table 3 show that the lung index of mice infected with influenza A (H1N1) virus PR8 strain virus was significantly increased, which was significantly different from that of the normal control group (P<0.01). After 4 days of treatment with Compound 4 starting on the day of infection, the 15 mg/kg/d dose group of Compound 4 significantly reduced the lung index of mice after infection, with a significant difference compared with the model control group (P<0.01), lung index inhibition rate is 54.04%, and the inhibition rate of oseltamivir at a dose of 27.5 mg/kg/d was 58.89%.

**Example 15: Therapeutic effects of compounds of the present invention on a mouse model of pneumonia infected with human coronavirus 229E**

**1 Experimental materials**

**1.1** Tested drugs: Compound 4, Compound F1, Compound F2, Compound F3

**1.2** Positive control drugs: Chloroquine phosphate tablets, Sichuan Shenghe Pharmaceutical Co. Specification: 0.25g/ tablet, Dosage: 0.5 g/60kg/d, orally.

**1.3** Test animals

| Strain | level | weight | Number | gender | Certificate of Conformity number |
|---|---|---|---|---|---|
| BALB/c mouse | SPF grade | 13~15 | 110 | 50/50 male and female | 1100112011018144 |
| license number | SCXK(jing)2016-0009 | | | | |
| Animal source | Beijing Viton Lihua Laboratory Animal Technology Co. | | | | |

**1.4** Strains and cells: Human Coronavirus 229E (HCoV-229E), was provided by the Institute of Pharmaceutical Biotechnology of the Chinese Academy of Medical Sciences, which was passaged in our laboratory and stored in -80°C refrigerator. Human embryonic lung fibroblasts MRC5, was purchased from Beijing Beina Chuanglian Biotechnology Research Institute, which was passaged in our laboratory and stored in liquid nitrogen.

**1.5** Test instruments

| Instrument Name | model number | manufacturer | Instrument Usage |
|---|---|---|---|
| A2 type biological safety cabinet | Thermo MSC1.8 | Thermo Corporation, USA | Animal infections |
| Electronic balance | YP1002 | Shanghai Yue Ping Scientific Instrument Co. | Weighing of mice |
| Electronic balance | AL204 | METTLER TOLEDO INSTRUMENTS | Weighing tissue weight |
| IVC Mouse Cages | ZJ-4 | Suzhou Fung Co. | Mouse feeding |

**2 Test Methods**

**2.1** Dosage design and formulation

Drug on test

| | |
|---|---|
| Mouse dosage | Doses of 50mg/kg, 40mg/kg were used in the experiment. |
| Mouse dosa | 20ml/kg/d, gavage |
| Formulation concentration | The solutions were prepared into corresponding concentrations with CMC-Na respectively, and the mice were administered by gavage at 0.2 ml/10 g/d once a day during the test. |

Chloroquine phosphate tablets

| | |
|---|---|
| clinical dose | 0.5 g/60kg/d orally |
| Mouse dose | Clinical doses were converted to mouse doses: 0.5 g/60kg/d×11=0.09 g/kg/d |
| Dose given to mice | 20ml/kg/d, gavage |
| formulated concentration | 0.09 g/kg/d÷20ml/kg/d=0.0045 g/ml |
| Formulation method | 1 tablet (0.25 g) Dissolve in distilled water until → 56ml，4°C storage. |

**2.2** Virus Passage

A 25cm$^2$ culture flask with a monolayer of MRC-5 cells was taken, the culture medium was discarded, the cell surface was rinsed with cell maintenance solution for 3 times, 5ml of cell maintenance solution was added, and then 200μl of HCoV-229E virus solution was added. The cells were placed in 37°C 5% $CO_2$ incubator for 72~ 96h, and the cell lesions were observed under an inverted microscope every day until 80% of the cells showed obvious lesions (CPE), then the cell culture flask was placed in -80°C cryogenic refrigerator for freezing, and the viral solution was subjected to freezing-thawing for 3 times so as to be used for the determination of viral titer.

**2.3** Determination of viral titer

A 96-well plate with a monolayer of MRC5 cells was taken, the culture medium was discarded, the cells were rinsed with cell maintenance solution 3 times, diluted according to 10-fold multiplicity ($10^{-1}$~$10^{-8}$), a total of 8 dilutions, and inoculated with different titers of HCoV-229E virus solution at 100 μl/well (4 replicate wells for each dilution), and at the same time, a normal cell control was set up. 96-well plates were incubated at 37°C in a 5% $CO_2$ incubator for 72-96h, and the cytopathic condition was observed under an inverted microscope every day, and the cytopathic condition of each well

was recorded. The cell lesions in each well were recorded. The 50% cytopathic concentration ($TCID_{50}$) was calculated according to the Reed-Muench method.

**2.4** Construction and administration of a mouse model of human coronavirus pneumonia

BALB/c mice were taken and randomly divided into a normal control group, model control group, chloroquine phosphate control group, and two dosage groups of compound 4, compound F1, compound F2, and compound F3 for each drug, with 10 mice in each group, 50/50 male and female . Except the normal control group, the mice in each group were slightly anesthetized with ether, and then infected with 100$TCID_{50}$ HCoV-229E nasal drops, 50 μl/animal, once every other day, for a total of two infections. On the day of the initial infection, each dosing group was administered once a day for 4 consecutive days. The mice were weighed on the day after the last administration, and dissected. The lungs were weighed to calculate the lung index and inhibition rate of the mice.

$$\text{Lung index (\%)} = \text{wet lung weight (g)} \times 100/\text{ body weight (g)}$$

$$\text{Lung index inhibition rate (\%)} = \frac{\text{Lung weight in model control group (g)-} \\ \text{Lung weight of administered group (g)}}{\text{Lung weight in model control group (g)-} \\ \text{Normal control lung weight (g)}} \times 100\%$$

**3 Test results**

**Table 4 Effects of compounds of the present invention on a mouse model of pneumonia infected by human coronavirus 229E**

| groups | dosage (mg/kg/d ) | numb er of anima ls (n) | lung index(g lung weight × 100/g body weight) | inhibitio n rate (%) |
|---|---|---|---|---|
| Normal control group | - | 10 | 0.64±0.05 | - |
| Model Control Group | - | 10 | $0.85\pm0.07^{\#\#}$ | - |
| Chloroquine control group | 90 | 10 | 0.78±0.04* | 31.34 |
| Compound 4 | 50 | 10 | 0.76±0.08* | 41.00 |
| | 40 | 10 | 0.76±0.02* | 41.85 |
| Compound F1 | 50 | 10 | 0.80±0.05 | 20.36 |
| | 40 | 10 | 0.81±0.06 | 15.89 |
| Compound F2 | 50 | 10 | 0.82±0.06 | 10.58 |
| | 40 | 10 | 0.84±0.05 | 1.45 |
| Compound F3 | 50 | 10 | 0.84±0.06 | 1.81 |
| | 40 | 10 | 0.83±0.09 | 7.71 |

Note: Comparison with normal group P<0.01; Comparison with model control group, *P<0.01

The results in Table 4 show that after being infected with human coronavirus 229E, the lung index of mice in the model control group increased significantly, and there was a significant difference (P<0.01) compared with the normal control group. Compound 4 significantly decreased the lung index of mice in the dose groups of 50mg/kg and 40mg/kg, with significant difference (P<0.05) compared with the model control group, and the inhibition rates were 41.00% and 41.85%, respectively. Compound F1, compound F2 and compound F3 had no significant effects on the lung index of mice. Compound 4 was more potent than compound F1, indicating that the 7-position substituent has a very important effect on the anticoronaviral potency of such isoflavone derivatives. Compound 4 was more potent than compounds F2 and F3, suggesting that the position and structure of the substituent group have an important influence on the anticoronaviral efficacy of these isoflavone derivatives.

**Example 16: Effects of the compounds of the present invention on a mouse pneumonia model of coronavirus 229E infection**

Test reagents, test apparatus, test methods are described as above.

Tested samples: Compound 4, Compound F4, Compound F5, Compound 5, Compound 7

Certificate of Animal Conformity: BALB/c mice110011201106656 2881/718

**Table 5 Effects of compounds of the present invention on a mouse pneumonia model of coronavirus 229E infection**

| groups | dosage (mg/kg/ d) | number of animals (n) | lung index(g lung weight × 100/g body weight) | inhibition rate (%) |
|---|---|---|---|---|
| Normal control group | - | 10 | 0.67±0.05 | - |
| Model Control Group | - | 10 | 0.76±0.06## | - |
| Chloroquin e control group | 90 | 10 | 0.70±0.04** | 56.24 |
| Compound 4 | 30 | 10 | 0.66±0.05** | 86.46 |
| | 15 | 10 | 0.67±0.08** | 76.52 |
| | 7.5 | 10 | 0.71±0.11** | 44.34 |
| Compound F4 | 30 | 10 | 0.75±0.13 | 37.23 |
| | 15 | 10 | 0.84±0.07 | -64.00 |
| | 7.5 | 10 | 0.86±0.07 | -86.00 |
| Compound F5 | 30 | 10 | 0.77±0.11 | 16.96 |
| | 15 | 10 | 0.85±0.08 | -72.00 |
| | 7.5 | 10 | 0.82±0.08 | -41.00 |
| Compound 5 | 30 | 10 | 0.79±0.08 | -7.00 |
| | 15 | 10 | 0.80±0.10 | -17.00 |
| | 7.5 | 10 | 0.76±0.08 | 22.69 |
| Compound 7 | 30 | 10 | 0.76±0.07 | 15.76 |
| | 15 | 10 | 0.74±0.06* | 41.56 |
| | 7.5 | 10 | 0.80±0.09 | -17.00 |
| Note: Comparison with normal group ## P<0.01; Comparison with model control group, **P<0.01, *P<0.05 | | | | |

The results in Table 5 show that the lung index of mice infected with human coronavirus 229E was significantly increased, which was significantly different from that of the normal control group (P<0.01). After 4 days of treatment with Compounds of the present invention starting on the day of infection, Compound 4 at a dose of 30mg/kg/d, 15mg/kg/d, 7.5mg/kg/d significantly reduced the lung index of mice after infection, with a significant difference compared with the model control group (P<0.0 1, P<0.05), and lung index inhibition was 86.46%, 76.52%, and 44.34%, respectively. Compound 7 at a dose of 15mg/kg/d significantly reduced the lung index of mice after infection, with a significant difference compared with the model control group (P<0.05), and lung index inhibition was 41.56%. Compound F4, compound F5 and compound 5 had no significant effects on the lung index of mice. Compound F4, compound F5, compound 5, and compound 7 showed decreasing potency relative to compound 4, proving that the 2-(cyclopropylamino)ethoxy substituent at position 7 has a very important role in influencing the anticoronaviral potency of such isoflavone derivatives.

**Example 17: Therapeutic effects of compound 4 on a mouse pneumonia model of human coronavirus OC43 infection**

Test reagents, test apparatus, test methods as above.

Tested sample: Compound 4

Certificate of Animal Conformity: BALB/c mice110011211110038632

**Table 6 Effects of compound 4 of the present invention on human coronavirus OC43-infected mouse pneumonia model**

| groups | dosage (mg/kg/d ) | numb er of anima ls (n) | lung index(g lung weight × 100/g body weight) | inhibitio n rate (%) |
|---|---|---|---|---|
| Normal control group | - | 10 | 0.69±0.06 | - |
| Model Control Group | - | 10 | 0.87±0.09## | - |
| Chloroquine control group | 90 | 10 | 0.80±0.05* | 42.72 |
| Compound 4 | 15 | 10 | 0.79±0.10* | 47.43 |
| | 7.5 | 10 | 0.82±0.05 | 32.42 |
| | 3.75 | 10 | 0.81±0.08 | 33.28 |

Note: Comparison with normal group ## P<0.01; Comparison with model control group, *P<0.01

The results in Table 6 show that the lung index of mice infected with human coronavirus OC43 was significantly increased, which was significantly different from that of the normal control group (P<0.01). After 4 days of treatment with Compound 4 starting on the day of infection, Compound 4 at a dose of 15mg/kg/d significantly reduced the lung index of mice after infection, with a significant difference compared with the model control group (P<0.05), and lung index inhibition was 47.43%, and the inhibition rate of chloroquine phosphate at a dose of 90 mg/kg/d was 42.72%.

**Example 18: Effects of compound 4 of the present invention on a mouse pneumonia model of parainfluenza virus infection**

**1 Experimental materials**

**1.1** Tested drugs: Compound 4

**1.2** Ribavirin Granules Manufactured by Sichuan Baili Pharmaceutical Co.

Indications: This product is suitable for viral pneumonia, bronchitis, and skin herpes virus infection caused by respiratory syncytial virus. Main components: ribavirin. Properties: White or off-white soluble granules. Specification: 50mg/bag, 18 bags/box. Usage and dosage: Oral, 150mg once, 3 times a day. Storage conditions: airtight, stored in a dry place.

**1.3** Test animals

| strain | lev el | weigh t (g) | Num ber (n) | gen der | Certifies te of Conform ity number | Certific ate of Confor mity number | Source |
|---|---|---|---|---|---|---|---|
| ICR mouse | SP F gra de | 13~15 | 85 | 50/5 0 mal e and fem ale | 11001121 11024700 23 | SCXK(ji ng) 2016 -0006 | Beijing Viton Lihua Laboratory Animal Technology Co. |

**1.4** Virus strains: Parainfluenza virus, purchased from ATCC, which was stored at -80°C in this research laboratory.

**1.5** Test instruments

| Instrument Name | model number | manufacturer | Instrument Usage |
|---|---|---|---|
| A2 type biological safety cabinet | Thermo MSC1.8 | Thermo Corporation, USA | Animal infections, anatomy |
| Electronic balance | YP1002 MAX100g | Shanghai Yue Ping Scientific Instrument Co. | Weighing of mice |
| Electronic balance | AL204 MAX210g | METTLER TOLEDO INSTRUMENTS | Weighing tissue weight |
| IVC Mouse Cages | ZJ-4 | Suzhou Fung Co. | Mouse feeding |

**2 Test Methods**

**2.1** Dosage design and formulation

Drug on test

| | |
|---|---|
| Compond 4 | The experimental dosage for mice was: 15mg/kg/d, 7.5mg/kg/d, 3.75mg/kg/d |
| Mouse dosage | 20ml/kg/d, gavage |
| Formulation concentration | The solutions were prepared into corresponding concentrations with CMC-Na respectively, and the mice were administered by gavage at 0.2 ml/10 g/d once a day during the test. |

Ribavirin granules

| | |
|---|---|
| Human clinical dose | 450mg/60kg/d |
| Mouse dose | Clinical doses were converted to mouse doses:450mg/60kg/d×11=82.5mg/kg/d |
| Dose given to mice | 20ml/kg/d, gavage |
| formulated concentration | 82.5mg/kg/d÷20ml/kg/d=4.125mg/ml |

**2.2** Modeling and drug administration

Sixty ICR mice (SPF grade, weighing 13-15 g) were taken and randomly divided into six groups according to the body weight: a normal control group, model control group, ribavirin group, and three dosage groups of Compound 4, 10 mice in each group, 50/50 male and female. Except the normal control group, mice in all groups were slightly anesthetized with ether and then infected with 100$TCID_{50}$ Sendai strain of parainfluenza virus by nasal drip at 45 μl per mouse. Mice in each group were given an appropriate drug after infection, and distilled water was given to the normal control group and model control group under the same conditions for 4 consecutive days. On Day 5, the mice were weighed and the lungs were isolated and weighed.

$$\text{Lung index} = \text{lung weight x } 100/\text{body weight}$$

$$\text{Lung index inhibition rate (\%)} = \frac{\text{Lung weight in model control group (g)} - \text{Lung weight of administered group (g)}}{\text{Lung weight in model control group (g)} - \text{Normal control lung weight (g)}} \times 100\%$$

**Table 7 Effects of Compound 4 on Parainfluenza Virus Infected Mouse Pneumonia Model**

| Groups | dosage (mg/kg/ d) | number of animals (n) | lung index(lung weight/100 g body weight) | inhibition rate (%) | death |
|---|---|---|---|---|---|
| Normal control group | - | 10 | 0.63±0.04 | - | / |
| Model Control Group | - | 10 | 0.75±0.06## | - | / |
| Ribavirin | 82.5 | 10 | 0.70±0.03* | 42.06 | |
| Compound 4 | 15 | 10 | 0.70±0.05* | 46.39 | / |
| | 7.5 | 10 | 0.72±0.05 | 26.20 | |
| | 3.75 | 10 | 0.73±0.07 | 18.11 | |
| Note: Comparison with normal group ## P<0.01; Comparison with model control group, *P<0.05 | | | | | |

The results in Table 7 showed that: after being infected by parainfluenza virus, the lung index of mice in the model control group increased significantly, and there was a significant difference compared with the normal control group (P<0.01). The lung index of mice in the 15 mg/kg dose group of Compound 4 was significantly reduced, and there was a significant difference compared with the model control group (P<0.05). There was a good quantity-effect correlation among the three dose groups, and the inhibition rate of the high-dose group of Compound 4 was 46.39%.

**Example 19: Compound 4 of the present invention against novel coronavirus (SARS-CoV-2) *in vitro***

1 Experimental materials

1.1 Tested drugs: Compound 4, Compound F1, Compound F2, Compound F3

1.2 Cell line: VeroE6 cells, preserved in the Virus Room of the State Key Laboratory of Respiratory Diseases, Guangzhou Institute of Respiratory Health.

1.3 Virus strain: SARS-CoV-2, with a titer of TCID50 = $10^{-6}$/100 μL, stored at -80°C by the National Key Laboratory for Biosafety Testing (P3 Laboratory) of the Institute of Hygiene and Quarantine, Guangzhou Customs Technology Center; the titer of the virus used was 100 TCID50.

**2 Test Methods**

2.1 Sample preparation

Drug name, experimental concentration and group

| group | | Drug concentration (μg/mL) |
|---|---|---|
| Experimental group 1 | Compound 4 | 15.6, 7.8, 3.9, 1.95 |
| virus control group | SARS-CoV-2 Virus Liquid | 100 $TCID_{50}$/ 孔 |
| cell control | culture medium | |

2.2 Antiviral assay of the test drug

① Sterile 96-well culture plate, 100 μL of VeroE6 cells at a concentration of 2 × $10^5$ cells/mL were added to each well, and incubated at 37 °C,5% $CO_2$ for 24 hours;

(2) To the experimental group of culture plate and virus control group were added 100 $TCID_{50}$ virus solution at 100 μL/well and incubated for 2 h in a 37°C, 5% $CO_2$ incubator;

③After 2 h, the cell culture solution in 96-well culture plate was discarded. The tested drugs were diluted into each concentration in Table 1 (3 replicate wells for each concentration), and the drug solutions were added at 100 μl/well;

(4) A cell control, a blank control (solvent control), and a virus control (negative control) were also set up;

⑤ Cells were incubated in a 37°C, 5% $CO_2$ incubator for 3~4 days;

⑥ Cytopathic lesions (CPE) were observed under an optical microscope, and the cytopathic lesion extent of the cells was recorded according to the following six levels of criteria: "-" no lesions; "±" was less than 10% of the cytopathic lesion; "+" is about 25% of the cytopathic lesion; '++' is about 50% of the cytopathic lesion; '+++' is about 75% of the cytopathic lesion; " ++++" is more than 75% of the cytopathic lesions. The half effective concentration ($IC_{50}$) was calculated using the Reed-Muench method or GraphPad Prism 5.0. Criteria for determining efficacy: concentrations with 50% inhibition of viral CPE were considered as effective concentrations.

**3 Test results**

The cytopathic lesions (CPE) was observed, the experimental results were recorded, and the half effective concentration ($IC_{50}$) was calculated using the Reed-Muench method or GraphPad Prism 5.0. The results are listed as follows

**Table 8 Anti novel coronavirus efficacy results of Compound 4**

| Drug concentration (μg/mL) | Inhibition rate (%) |
|---|---|
| 15.6 | 90.00±5.00 |
| 7.8 | 76.67±2.89 |
| 3.9 | 25.00±5.00 |
| 1.95 | 6.67±2.89 |

The results of the efficacy assay of Compound 4 against the novel coronavirus are shown in Figure 1.
The results in Table 8 show that the drug, Compound 4 inhibited the cytopathic effect of novel coronavirus (SARS-CoV-2) infection on Vero E6 cells *in vitro.*

**Example 20: Study of the mechanism of action of compound 4 of the present invention against SARS-Cov-2 *in vitro***

**1. Binding effect on ACE2 receptor of 293T cells**

**Aim of the test:** To observe the binding effect of the tested samples to the ACE-2 receptor and to search for a possible mechanism of action.

**Instruments and Materials**

Tested drugs: Compound 4, Compound F1, Compound F2, Compound F3

SHIMADZU LC-2040C 3D High Performance Liquid Chromatograph (Japan); Sartorius 1712 Electronic Analytical Balance; Fulham FCR1002-UF Ultra Pure Water Preparation Machine (Qingdao Fulham Science & Technology Co., Ltd.); SHZ-D (III) Circulating Water Vacuum Pump (Gongyi Yingyu Yu Hua Instrument Factory); SB-5200DTD Ultrasonic

Cleaner ( Ningbo Xinzhi Bio-technology Co., Ltd.); pipette (Eppendorf, Germany).

293T cell line with high expression of ACE2 receptor was constructed in house.

Methanol is chromatographically pure, water is ultrapure, and all other reagents are analytically pure.

**Test Methods**

Sample binding effect with ACE2 receptor in 293T cells

Sample Preparation

5 mg of Compound 4, Compound F1, Compound F2 and Compound F3 of the present invention were precisely weighed respectively, fully dissolved in chromatographically pure methanol and transferred to a 10 mL measuring flask, and then diluted to the constant volume so as to obtain the stock solution of the compounds of the present invention at a concentration of 0.5 mg/mL, and then kept under refrigeration and in darkness for future use. When using it, it was diluted to the required concentration, and the stock solution will be freshly prepared every week.

CMC chromatographic conditions

ACE2-293T/CMC cell membrane chromatography column (10 × 2.0 mm), mobile phase was water, flow rate 0.2 mL/min, column temperature was 37 °C, injection volume was 10 μL, DAD detection. Positive control: chloroquine phosphate.

**Test results**

The cell membrane chromatograms of the compounds of the present invention are shown in Figure 2.

**Table 9 KD values of samples and chloroquine phosphate**

| name | Retention time/(min) | Capacity factor ks | KD value/(mol/L) |
|---|---|---|---|
| Compound 4 | 6.597 | 6.57 | $1.07\times10^{-6}$ |
| Compound F1 | - | - | - |
| Compound F2 | - | - | - |
| Compound F3 | - | - | - |

**2. Evaluation of the effects of compound 4 on the function of ACE2-293T cells**

**Experimental method:** intracellular Ca2+ flow change detection: cells were inoculated into 96-well plates, kept overnight, and then the medium was discarded. The cells were washed with CIB, Fluo-3 was added, and the AM incubation solution was incubated in the incubator for 40 min and then discarded. The cells were washed with CIB, and under a fluorescence microscope, CIB was discarded and then drugs were added sequentially in each well to observe the changes in cell fluorescence intensity before and after drug addition. Under the fluorescence microscope, the cells were continuously observed for 2 min, and the drug was added for stimulation at 10 s after the start of photo observation.

**RESULTS:** Changes in intracellular $Ca^{2+}$ flow: Compound 4 stimulates calcium mobilization in ACE2-293T cells at 10 μg/mL, and the degree of intracellular calcium fluctuation in ACE2-293T cells was dose-dependently enhanced with increasing concentrations of the administered drug. The calcium imaging results of Compound 4 in ACE2-293T cells are plotted in Figure 3.

**Example 21: Effects of Compound 4 on Inflammatory Factor Levels in Lung Tissue of Influenza Virus-Infected Mice**

Lung tissue samples: therapeutic effects of Compound 4 on a mouse pneumonia model infected with influenza virus strain H1N1/ FM1

**Test Method:** Mouse Lung Tissue Inflammatory Factor Assay (Elisa)

Tissue homogenate samples: Mouse lung tissues were taken and weighed, collected and stored at -4°C. After 50 mg of lung tissue was weighed and added into 500 μL of saline, the tissue was homogenized using an ultrasonic cell

crusher, and centrifuged at -4°C for 10 minutes at 1000 × g using a low-temperature high-speed centrifuge. The supernatant was aspirated and dispensed and stored at -80°C in a refrigerator. Repeated freezing and thawing shall be avoided.

Sample preparation: Samples were tested after 2-fold dilution with diluent, i.e., 50 μL serum + 50 μL diluent.

The microtiter plate was taken from the sealed bag that has been equilibrated to room temperature and different concentrations of standards, samples or QCs were added to the corresponding wells at 100 μL per well. The wells were sealed with sealing tape and incubated for 2 hours at room temperature. The plate was washed with a washing solution for 4 times. At the end of the last wash, the plate was inverted and patted to dry any residual liquid on an absorbent paper; 100 μL of ELISA antibody was added to each micro-well. The reaction wells were sealed with sealing tape and incubated for 2 hours at room temperature. Step 4 was repeated, 100 μL of color development substrate was added to each well and incubated for 30 minutes at room temperature in darkness. 100 μL of quenching solution was added to each well, and then the absorbance was measured at 450 nm using an enzyme meter within 30 minutes of adding, and the results were calculated.

The results of Figures 4 and 5 showed that after infection of mice with influenza virus H1N1/FM1 strains, the IFN-β and TNF-a contents in the lung tissues of mice were increased, with significant differences compared with the normal control group ($p<0.01$); Compound 4, after 4 days of administration, significantly reduced the IFN-β and TNF-a contents in the lung tissues of mice after infection, with significant differences compared with the model control group ($p <0.01$).

**Example 22: Effects of Compound 4 on Inflammatory Factor Levels in Lung Tissue of Coronavirus 229E-Infected Mice**

**Lung tissue samples:** therapeutic effects of Compound 4 on a mouse pneumonia model infected with human coronavirus 229E

The results of Figures 6 and 7 showed that after being infected by human coronavirus 229E, the IL-1 and IL-10 content in the lung tissues of mice increased, with a significant difference compared with the normal control group ($p<0.01$); Compound 4, after 4 days of administration, significantly reduced the IL-1 and IL-10 content in the lung tissues of infected mice, with a significant difference compared with the model control group ($p<0.01$).

**Example 23 Therapeutic effects of compounds of the present invention on a mouse pneumonia model infected with human coronavirus 229E**

**1 Experimental materials**

**1.1** Tested drugs: Compound 4, Compound 9, Compound 10, Compound 11, Compound F1.

**1.2** Positive control drugs: Chloroquine phosphate tablets, Sichuan Shenghe Pharmaceutical Co. Specification: 0.25g/tablet, Dosage: 0.5 g/60kg/d, orally.

**1.3** test animals

| strain | level | weig ht | Num ber | gend er | Certificate of Conformity number | license number |
|---|---|---|---|---|---|---|
| BALB/c mouse | SPF grade | 13~1 5 | 80 | 50/5 0 male and fema le | No.11001121111192 0584<br>No.11001121111192 0436 | SCXK(jin g) 2019-000 9 |
| Source | Beijing Viton Lihua Laboratory Animal Technology Co. | | | | | |

**1.4** Strains and cells: Human Coronavirus 229E (HCoV-229E), was provided by the Institute of Pharmaceutical Biotechnology of the Chinese Academy of Medical Sciences, which was passaged in our laboratory and stored in -80°C refrigerator. Human embryonic lung fibroblasts MRC5, was purchased from Beijing Beina Chuanglian Biotechnology Research Institute, which was passaged in our laboratory and stored in liquid nitrogen.

**1.5** Test instruments

| Instrument Name | model number | manufacturer | Instrument Usage |
|---|---|---|---|
| A2 type biological safety cabinet | Thermo MSC1.8 | Thermo Corporation, USA | Animal infections |
| Electronic balance | YP1002 | Shanghai Yue Ping Scientific Instrument Co. | Weighing of mice |
| Electronic balance | AL204 | METTLER TOLEDO INSTRUMENTS | Weighing tissue weight |
| IVC Mouse Cages | ZJ-4 | Suzhou Fung Co. | Mouse feeding |

**2 Test Methods**

**2.1** Dosage design and formulation

Drug on test

| | |
|---|---|
| A dose of 7.5 mg/kg/d was used for each drug in the trial | |
| Mouse dose | 20ml/kg/d, gavage |
| Formulation concentration | The solutions were prepared into corresponding concentrations with CMC-Na respectively, and the mice were administered by gavage at 0.2 ml/10 g/d once a day during the test. |

Chloroquine phosphate tablets

| | |
|---|---|
| clinical dose | 0.5 g/60kg/d orally |
| Mouse dose | Clinical doses were converted to mouse doses: 0.5 g/60kg/d×11=0.09 g/kg/d |
| Dose given to mice | 20ml/kg/d, gavage |
| formulated concentration | 0.09 g/kg/d÷20ml/kg/d=0.0045 g/ml |
| Formulation method | 1 tablet (0.25 g) Dissolve in distilled water until → 56ml， 4°C storage. |

**2.2** Virus Passage

A 25cm$^2$ culture flask with a monolayer of MRC-5 cells was taken, the culture medium was discarded, the cell surface was rinsed with a cell maintenance solution for 3 times, 5ml of cell maintenance solution was added, and then 200μl of HCoV-229E virus solution was added. The cells were placed in a 37°C 5% $CO_2$ incubator for 72~ 96h, and the cell lesions were observed under an inverted microscope every day until 80% of the cells showed obvious lesions (CPE). And then the cell culture flask was placed in a -80°C cryogenic refrigerator for freezing, and the viral solution was subjected to freezing-thawing for 3 times for the determination of viral titer.

**2.3** Determination of viral titer

A 96-well plate with a monolayer of MRC5 cells was taken, the culture medium was discarded, and the cells were

rinsed with a cell maintenance solution for 3 times, diluted according to 10-fold multiplicity ($10^{-1}$~$10^{-8}$), a total of 8 dilutions and inoculated with different titers of HCoV-229E virus solution at 100 μl/well (4 replicate wells for each dilution), and at the same time, a normal cell control was set up. 96-well plates were incubated at 37 °C in a 5% $CO_2$ incubator for 72-96h, and the cytopathic condition was observed under an inverted microscope every day, and the cytopathic condition of each well was recorded. The cell lesions in each well were recorded. The 50% cytopathic concentration ($TCID_{50}$) was calculated according to the Reed-Muench method.

**2.4** Construction and administration of a mouse model of human coronavirus pneumonia

BALB/c mice were taken and randomly divided into a normal control group, model control group, chloroquine phosphate control group, and one dosage group of compound 4, compound 9, compound 10, and compound 11 respectively, with 10 mice in each group, 50/50 male and female. Except the normal control group, the mice in each group were slightly anesthetized with ether, and then infected with $100TCID_{50}$ HCoV-229E nasal drops, 50 μl/animal, once every other day, for a total of two infections. On the day of the initial infection, each dosing group was administered once a day for 4 consecutive days. The mice were weighed on the day after the last administration, and dissected. The lungs were weighed to calculate the lung index and inhibition rate of the mice.

$$\text{Lung index (\%)} = \text{wet lung weight (g)} \times 100/\ \text{body weight (g)}$$

$$\text{Lung index inhibition rate (\%)} = \frac{\text{Lung weight in model control group (g)-}\ \text{Lung weight of administered group (g)}}{\text{Lung weight in model control group (g)-}\ \text{Normal control lung weight (g)}} \times 100\%$$

3 Test results

**Table 10 Effects of compounds of the present invention on a mouse pneumonia model of infected by human coronavirus 229E**

| groups | dosage (mg/kg/ d) | number of animals (n) | lung index(g lung weight × 100/g body weight) | inhibiti on rate (%) |
|---|---|---|---|---|
| Normal control group | - | 10 | 0.72±0.05 | |
| Model Control Group | CMC | 10 | 0.89±0.11# | |
| Chloroquine | 90 | 10 | 0.80±0.02* | 52.88 |
| Compound 4 | 7.5 | 10 | 0.81±0.03* | 50.04 |
| Compound 9 | 7.5 | 10 | 0.81±0.10* | 46.45 |
| Compound 10 | 7.5 | 10 | 0.85±0.06 | 25.56 |
| Compound 11 | 7.5 | 10 | 0.87±0.08 | 14.06 |
| Compound F1 | 7.5 | 10 | 0.85±0.07 | 27.22 |

Note: Comparison with normal group ## P<0.05; Comparison with model control group, *P<0.05

The results in Table 10 show that after being infected with human coronavirus 229E, the lung index of mice in the model control group increased significantly, and there was a significant difference (P<0.05) compared with the normal control group. Compound 4 and compound 9 significantly decreased the lung index of mice in the dose groups of 7.5mg/kg, with significant difference (P<0.05) compared with the model control group.

**Example 24: Effects of compounds of the present invention on a mouse pneumonia model of parainfluenza virus infection**

**1 Experimental materials**

**1.1** Tested drugs: Compound 4, Compound 9, Compound 10, Compound 11, Compound 12, Compound 2

**1.2** Ribavirin Granules Manufactured by Sichuan Baili Pharmaceutical Co. Indications: This product is suitable for viral pneumonia, bronchitis, and skin herpes virus infection caused by respiratory syncytial virus. Main components: ribavirin. Properties: White or off-white soluble granules. Specification: 50mg/bag, 18 bags/box. Usage and dosage: Oral, 150mg once, 3 times a day. Storage conditions: airtight, stored in a dry place.

**1.3** Test animals

| strain | lev el | weigh t (g) | Num ber (n) | gen der | Certifies te of Conform ity number | Certific ate of Confor mity number | Source |
|---|---|---|---|---|---|---|---|
| ICR mouse | SP F gra de | 13~15 | 150 | 50/5 0 mal e and fem ale | No.11001 12111129 45274<br>No.11001 12111129 45145 | SCXK(ji ng) 2016 -0006 | Beijing Viton Lihua Laboratory Animal Technology Co. |

**1.4** Virus strains: Parainfluenza virus, purchased from ATCC, which was stored at -80°C in this research laboratory.

**1.5** Test instruments

| Instrument Name | model number | manufacturer | Instrument Usage |
|---|---|---|---|
| A2 type biological safety cabinet | Thermo MSC1.8 | Thermo Corporation, USA | Animal infections, anatomy |
| Electronic balance | YP1002 MAX100g | Shanghai Yue Ping Scientific Instrument Co. | Weighing of mice |
| Electronic balance | AL204 MAX210g | METTLER TOLEDO INSTRUMENTS | Weighing tissue weight |
| IVC Mouse Cages | ZJ-4 | Suzhou Fung Co. | Mouse feeding |

**1.6** Test site: ABSL-2 Laboratory, Institute of Traditional Chinese Medicine, China Academy of Traditional Chinese Medicine, China

**2 Test Methods**

**2.1** Dosage design and formulation

Drug on test

| | |
|---|---|
| Administration dose | The experimental dosage for mice was: 15mg/kg/d, 7.5mg/kg/d, 3.75mg/kg/d |

(continued)

| | |
|---|---|
| Mouse dosage | 20ml/kg/d, gavage |
| Formulation concentration | The solutions were prepared into corresponding concentrations with CMC-Na respectively, and the mice were administered by gavage at 0.2 ml/10 g/d once a day during the test. |

Ribavirin granules

| | |
|---|---|
| Human clinical dose | 450mg/60kg/d |
| Mouse dose | Clinical doses were converted to mouse doses:450mg/60kg/d×11=82.5mg/kg/d |
| Dose given to mice | 20ml/kg/d, gavage |
| formulated concentration | 82.5mg/kg/d÷20ml/kg/d=4.125mg/ml |

2.2 Modeling and drug administration

Sixty ICR mice, (SPF grade, weighing 13-15 g), were taken and randomly divided into fifteen groups according to body weight: a normal control group, model control group, ribavirin group, and two dosage groups of compound 4, compound 9, compound 10, compound 11, compound 12 and compound 2, 10 mice in each group, 50/50 male and female. Except the normal control group, mice in all groups were slightly anesthetized with ether and then infected with $100TCID_{50}$ Sendai strain of parainfluenza virus by nasal drip at 45 μl per mouse. Mice in each group were given an appropriate drug after infection, and distilled water was given to the normal control group and model control group under the same conditions for 4 consecutive days. On Day 5, the mice were weighed and the lungs were isolated and weighed.

$$\text{Lung index} = \text{lung weight x 100/body weight}$$

$$\text{Lung index inhibition rate (\%)} = \frac{\text{Lung weight in model control group (g)-}\ \text{Lung weight of administered group (g)}}{\text{Lung weight in model control group (g)-}\ \text{Normal control lung weight (g)}} \times 100\%$$

**Table 11 Effects of the compounds of the present invention on a parainfluenza virus-infected mouse pneumonia model**

| groups | dosage (mg/kg/d) | number of animals (n) | lung index (Lung weight * 100/body weight) | inhibiti on rate % |
|---|---|---|---|---|
| Normal control group | - | 10 | 0.71±0.05 | - |
| Model Control Group | - | 10 | 0.84±0.09## | - |
| Ribavirin | 82.5 | 10 | 0.77±0.04* | 57.66 |
| Compound 4 | 15 | 10 | 0.78±0.04* | 45.45 |
| | 7.5 | 10 | 0.77±0.04* | 54.48 |
| Compound 9 | 15 | 10 | 0.77±0.05* | 55.59 |
| | 7.5 | 10 | 0.79±0.05* | 40.51 |
| Compound 10 | 15 | 10 | 0.86±0.05 | -16.30 |
| | 7.5 | 10 | 0.83±0.06 | 7.43 |

(continued)

| groups | dosage (mg/kg/d) | number of animals (n) | lung index | inhibiti on rate |
|---|---|---|---|---|
| | | | (Lung weight * 100/body weight) | % |
| Compound 11 | 15 | 10 | 0.83±0.05 | 6.19 |
| | 7.5 | 10 | 0.87±0.04 | -20.73 |
| Compound 12 | 15 | 10 | 0.76±0.03* | 61.91 |
| | 7.5 | 10 | 0.77±0.04* | 57.49 |
| Compound 2 | 15 | 10 | 0.84±0.07 | 3.27 |
| | 7.5 | 10 | 0.86±0.04 | -18.32 |
| Note: Comparison with normal group ## P<0.05; Comparison with model control group, *P<0.05 | | | | |

The results in Table 11 showed that: after being infected by parainfluenza virus, the lung index of mice in the model control group increased significantly, and there was a significant difference compared with the normal control group (P<0.01). Lung index of mice was significantly reduced in the 15mg/kg/d and 7.5mg/kg/d dose groups of Compound 4, Compound 9, and Compound 12, and there was a significant difference compared with the model control group (P<0.05).

**Example 25 Therapeutic effects of the compounds of the present invention on** a **mouse pneumonia model infected with influenza A virus H1N1/PR8, H1N1/FM1**

**1 Experimental materials**

**1.1** Tested drugs: Compound 9, Compound 12, Compound 2

**1.2** Positive control drugs

Oseltamivir phosphate capsule (Tamiflu) Manufactured by: Hoffmann · Roche Ltd, Basel, Switzerland. Indications: For the treatment of influenza A and B in adults and children aged 1 year and over; for the prophylaxis of influenza A and B in adults and adolescents aged 13 years and over. Main ingredient: Oseltamivir phosphate. Properties: The product is gray and light yellow capsule, the content is white to yellowish white powder, which may contain lumps. Specification: 75mg/capsule as oseltamivir, 10 capsules/box. Usage and dosage: Oral, adults and adolescents aged 13 years and above, 75mg each time, twice a day for 5 days. Storage condition: Keep sealed.

**1.3** Test animals

| strain | level | weight (g) | Number (n) | gender | Certificate of Conformity number |
|---|---|---|---|---|---|
| ICR mouse | SPF grade | 13-15 | 180 | 50/50 male and female | No.1100112111132445 71<br>No.1100112111132941 61<br>No.1100112111132942 17 |
| license number | SCXK(jing)2016-0006 | | | | |
| source | Beijing Viton Lihua Laboratory Animal Technology Co. | | | | |

**1.4** Virus strains

| Virus strain name | Source | Use |
|---|---|---|
| Influenza A (H1N1) virus PR8 strain, FM1 strain | ATCC | Construction of a model of pneumonia in mice infected with H1N1 influenza virus |

**1.5** Test instruments

| Instrument Name | Model number | Manufacturer | Instrument Usage |
|---|---|---|---|
| A2 type biological safety cabinet | Thermo MSC1.8 | Thermo Corporation, USA | Animal infections, anatomy |
| Electronic balance | YP1002 MAX100g | Shanghai Yue Ping Scientific Instrument Co. | Weighing of mice |
| Electronic balance | AL204 MAX210g | METTLER TOLEDO INSTRUMENTS | Weighing tissue weight |
| IVC Mouse Cages | ZJ-4 | Suzhou Fung Co. | Mouse feeding |

**1.6** Test site: ABSL-2 Laboratory, Institute of Traditional Chinese Medicine, China Academy of Traditional Chinese Medicine, China

**2 Test Methods**

**2.1** Dosage design and formulation

Drug on test

| | |
|---|---|
| experimental dose | Dosage for mice: 15mg/kg/d, 7.5mg/kg/d. |
| Mouse dosa | 20ml/kg/d, gavage |
| Formulation concentration | The solutions were prepared into corresponding concentrations with CMC-Na respectively, and the mice were administered by gavage at 0.2 ml/10 g/d once a day during the test. |

Oseltamivir phosphate capsule

| | |
|---|---|
| Human clinical dose | 150mg/60kg/d |
| Mouse dose | Clinical doses were converted to mouse doses: 150mg/60kg/d×11=27.5mg/kg/d |
| Mouse dosa | 20ml/kg/d, gavage |
| formulated concentration | 27.5mg/kg/d÷20ml/kg/d=1.375mg/ml |
| Formulation method | 1 grain (75mg) Dissolve in distilled water until → 55ml, 4°C storage. |

**2.2** test procedure

ICR mice were taken and randomly divided into 9 groups according to the body weight: a normal control group, model control group, oseltamivir control group, and two dosage groups of Compound 9, Compound 12 and Compound 2, 10 mice in each group. Except the normal control group, mice were slightly anesthetized with ether and infected with 15 $LD_{50}$ H1N1/PR8 influenza virus liquid by nasal drops at 30 μl per mouse. The administration was started on the day of infection, at 0.2 ml/10 g each time by gavage once a day for 4 days, and the normal control and model control groups were gavaged with distilled water under the same conditions. The mice were weighed and dissected on Day 5, and lungs were weighed to calculate lung index and lung index inhibition. The results were statistically processed using t-test for comparison between groups.

$$\text{Lung index (\%)} = \text{wet lung weight (g)} \times 100/\ \text{body weight (g)}$$

$$\text{Lung index inhibition rate (\%)} = \frac{\text{Lung weight in model control group (g)-}\ \text{Lung weight of administered group (g)}}{\text{Lung weight in model control group (g)-}\ \text{Normal control lung weight (g)}} \times 100\%$$

**3 Test results**

**Table 12 Therapeutic effects of compounds of the present invention on a mouse model of pneumonia infected with influenza virus strain H1N1/ PR8**

| groups | dosage (mg/kg/d) | number of animals (n) | lung index (Lung weight * 100/body weight) | inhibiti on rate % |
|---|---|---|---|---|
| Normal control group | - | 10 | 0.70±0.058 | - |
| Model Control Group | - | 10 | 0.81±0.101# | - |
| Tamiflu -positive drug group | 27.5 | 10 | 0.71±0.069* | 88.97 |
| Compound 9 | 15 | 10 | 0.71±0.060* | 91.64 |
| | 7.5 | 10 | 0.74±0.046* | 58.71 |
| Compound 12 | 15 | 10 | 0.72±0.076* | 76.54 |
| | 7.5 | 10 | 0.74±0.091* | 63.78 |
| Compound 2 | 15 | 10 | 0.74±0.091* | 60.90 |
| | 7.5 | 10 | 0.70±0.110** | 99.05 |
| Note: Comparison with normal group ## P<0.01; Comparison with model control group, **P<0.01, *P<0.05 | | | | |

The results in Table 12 show that the lung index of mice infected with influenza A (H1N1/PR8) virus was significantly increased, which was significantly different from that of the normal control group (P<0.01). Significant reduction in lung index was observed after 4 days of treatment with Compound 9, Compound 12, and Compound 2 starting on the day of infection, with a significant difference compared with the model control group (P<0.01).

**Table 13 Therapeutic effects of compounds of the present invention on a mouse pneumonia model infected with influenza virus strain H1N1/FM1**

| groups | dosage (mg/kg/d ) | number of animals (n) | lung index (Lung weight * 100/body weight) | inhibition rate % |
|---|---|---|---|---|
| Normal control group | - | 10 | 0.70±0.058 | - |
| Model Control Group | - | 10 | 0.80±0.077## | - |
| Tamiflu-positi ve drug group | 27.5 | 10 | 0.74±0.065* | 64.57 |
| Compound 9 | 15 | 10 | 0.71±0.052** | 84.63 |
| | 7.5 | 10 | 0.72±0.069* | 79.57 |
| Compound 12 | 15 | 10 | 0.71±0.042** | 93.59 |
| | 7.5 | 10 | 0.73±0.070* | 70.82 |
| Compound 2 | 15 | 10 | 0.73±0.060* | 72.90 |
| | 7.5 | 10 | 0.77±0.074 | 26.60 |
| Note: Comparison with normal group ## P<0.01; Comparison with model control group, **P<0.01, *P<0.05 | | | | |

The results in Table 13 show that significant increase in lung index in mice infected with influenza A (H1N1/FM1) viruses, which was significantly different from that of the normal control group (P<0.01). Significant reduction in lung index was observed after 4 days of treatment with Compound 9, Compound 12, and Compound 2 starting on the day of infection, with a significant difference compared with the model control group (P<0.01).

**Example 26: Therapeutic effects of compound 12 on a mouse pneumonia model infected with human coronavirus 229E**

The experimental materials and experimental methods were the same as those in Example 23.

**Table 14 Effects of Compound 12 on human coronavirus 229E infection in a mouse pneumonia model**

| groups | dosage (mg/kg/d) | number of animals (n) | lung index(g lung weight × 100/g body weight) | inhibiti on rate (%) |
|---|---|---|---|---|
| Normal control group | - | 10 | 0.78±0.04 | |
| Model Control Group | CMC | 10 | 0.91±0.06# | |
| Chloroquine | 90 | 7 | 0.83±0.05** | 62.80 |
| Compound 12 | 15 | 7 | 0.82±0.07** | 68.38 |
| | 7.5 | 7 | 0.86±0.04* | 39.78 |
| Note: Comparison with normal group ## P<0.05; Comparison with model control group, *P<0.05 | | | | |

The results in Table 14 show that after being infected with human coronavirus 229E, the lung index of mice in the model control group increased significantly, and there was a significant difference (P<0.05) compared with the normal control group. Compound 12 significantly decreased the lung index of mice in the dose groups of 15mg/kg and 7.5mg/kg, with significant difference (P<0.01, P<0.05) compared with the model control group.

**Example 27: Acute toxicity test in mice for Compound 4**

**1. Test methods**

Seventy ICR mice, weighing 17-21 g, were randomly divided into 7 groups according to fasting body weight: a normal control group and 6 dose groups of 475, 633, 844, 1125, 1500 and 2000 mg of the test drug (compound 4)/kg, 10 mice in each group, 50/50 male and female. After fasting for about 15 h, each dosing group was administered once by gavage in a volume of 40 ml/kg, and the control group was gavaged with an equal volume of 0.5% sodium carboxymethylcellulose solution once/day, and the animals were observed continuously for 14 days after administration of the

drug to observe the death of the animals.

Immediately after the administration of the drug, the animals were observed for the occurrence of toxic reactions, symptoms of toxicity and degrees thereof, the time of the appearance and disappearance of toxicity, the adverse reactions were recorded, and the dead animals were subjected to gross autopsy, including the observation of the animal's skin, eyes, ears, mouth, nose, genital pores, anus and its mucous membranes.

### 2. Experimental results

**2.1** Observation of mortality and adverse reactions in mice

Compound 4 was given to animals by gavage at doses of 475, 633, 844, 1125, 1500, and 2000 mg of test material/kg once/day, and the major toxic reaction observed was acute death.

Animal deaths occurred in the dose groups of 1125 mg drug/kg and above after administration of the drug, and no animal deaths occurred in the dose groups of 844 mg drug/kg and below, all of the animal deaths occurred within 4 d after the drug, and the peak of the deaths was within 24-48 h after the drug. The mortality rates of animals in the above six dose groups were 0%, 0%, 0%, 20%, 80% and 100%, respectively.

**2.2** In addition to acute death, other visible adverse reactions were mainly characterized by decreased spontaneous activity, sedentary lying, ataxia, tremor, convulsions, prone lying, loss of orthostatic reflexes, and erectile hairs; the above indications were altered to varying degrees in the 633, 844, 1125, 1500, and 2000 mg of drug/kg dose groups, with incidence rates of 40%, 60%, 80%, 100%, and 100%, which occurred within 60 min-5 d post-administration.No adverse visible reactions were observed in the 475 mg drug/kg dose group. Thereafter, until the end of the 14-day observation period, no abnormality was observed in the general condition, activity, gait, respiration, feeding, drinking, faeces and urine, as well as skin and fur of the animals.

In summary: The $LD_{50}$ was calculated by the Bliss method to be 1299.054 mg of drug/kg, with 95 % confidence limits ranging from 1152.937 to 1463.820 mg of drug/kg; the maximum non-lethal dose was 844 mg of drug/kg; and the maximum non-adverse effect dose was 475 mg of drug/kg.

### Example 28: Acute toxicity test in mice for Compound 12

### 1. Test Methods

Thirty ICR mice, weighing 17-21 g, were randomly divided into three groups according to their fasting body weight: a normal control group and 2000 mg and 4000 mg of test drug (Compound 12)/kg dose group, 10 mice in each group, 50/50 male and female. After fasting for about 15 h, each dosing group was given the drug once by gavage in a volume of 40 ml/kg, and the control group was given an equal volume of 0.5% sodium carboxymethylcellulose solution by gavage once/day, and the deaths of the animals were observed continuously for 14 days after the administration of the drug.

Immediately after the administration of the drug, the animals were observed for the occurrence of toxic reactions, symptoms of toxicity and degrees thereof, the time of the appearance and disappearance of toxicity, the adverse reactions were recorded, and the dead animals were subjected to gross autopsy, including the observation of the animal's skin, eyes, ears, mouth, nose, genital pores, anus and its mucous membranes.

### 2. Experimental results

Compound 12 was administered to the animals by gavage at a dose of 2000 and 4000 mg of the drug/kg once/day. The animals were observed immediately after administration and thereafter until the end of the observation period of 14 days, and no abnormality was observed in the general condition, activity, gait, respiration, feeding, drinking, faeces and urine, as well as skin and fur. No animals died during the experimental period.

In combination with the above embodiments, regarding the therapeutic effects of the compounds of the present invention on the mouse pneumonia model of influenza virus H1N1/ FM1 strain infection and the effects on the mouse pneumonia model of human coronavirus 229E infection, the efficacy of Compound 4 was more potent than that of the modification precursor 7-hydroxyisoflavone, which was not very potent, suggesting that the 7-position modification strategy of the present invention regarding 7-hydroxyisoflavone has a very important impact on the antiviral efficacy of antiviral efficacy of such isoflavone derivatives; and Compound 4 was more potent than Compound F2 (7-position glycosidylation strategy derivative), Compound F4 (7-position alkoxyalkoxy derivative), and Compound F5 (7-position amidoalkoxy derivative), indicating that the antiviral efficacy of Compound 4 obtained by the modification strategy of cycloalkylaminoalkoxy strategy was better. The antiviral potency of Compound 4 was more potent than both Compound 5 and Compound 7, indicating that the 7-position 2-(cyclopropylamino)ethoxy substituent derivative was more effective. It

indicates that the position of the substituent group as well as the structure has a very important influence on the antiviral potency of 7-hydroxyisoflavone derivatives, and it was experimentally demonstrated that the introduction of 2-(cyclopropylamino)ethoxy substituent at the 7-position had the best antiviral potency. Compound 9 and Compound 12 derived from compound 4 were also highly potent, but Compound 10 and Compound 11 derived from Compound 4 were less potent, indicating that the structure of the cyclopropyl group with $R_6$ in general formula (I) has a very important influence on the antiviral potency. In the case of retaining the cyclopropylamine substituent, the derivatives in which $R_6$ is an alkyl or substituted alkyl have strong pharmacological activity. Meanwhile, the results of the preliminary toxicology study showed that the $LD_{50}$ of Compound 4 was 1299.054 mg in the acute toxicity test in mice, and the maximum concentration of Compound 12 that could be administered by gavage was 4000 mg/kg, with no death of the animals, which indicated that the safety window of Compound 12 was larger and the efficacy of Compound 12 was better compared with Compound 4, indicating that the derivatives with $R_6$ being an alkyl or substituent alkyl group not only maintained a better pharmacological activity, but also maintain better pharmacological activity, and higher safety.

All documents referred to in this invention are cited as references in this application as if each document were cited individually as a reference. It is further to be understood that after reading the foregoing teachings of the present invention, those skilled in the art may make various alterations or modifications to the present invention, and these equivalent forms will likewise fall within the scope of the claims appended to this application.

## Claims

**1.** A compound shown in formula I, or a pharmaceutically acceptable salt, solvate, optically pure isomer, stereoisomer, or mixture thereof.

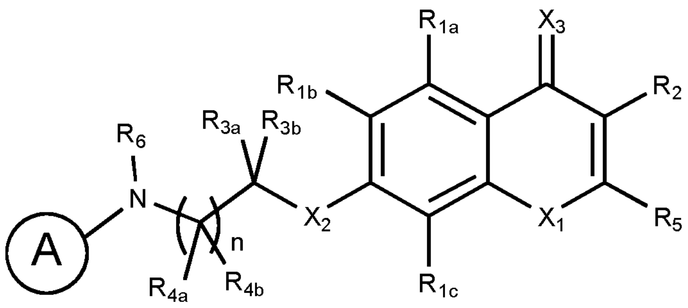

(I)

wherein $X_1$, $X_2$ are each independently selected from the group consisting of: O, $NR_7$; wherein said $R_7$ is selected from the group consisting of: a hydrogen, alkyl, substituted alkyl, arylalkyl, substituted arylalkyl, cycloalkyl, substituted cycloalkyl;

$X_3$ is selected from the following group: O, $NR_8$; wherein said $R_8$ is selected from the following group: hydrogen, alkyl;

$R_{1a}$, $R_{1b}$, $R_{1c}$ are each independently selected from the following group: a hydrogen, hydroxyl, alkoxy;

$R_2$ is selected from the following group: a substituted or unsubstituted aryl, or substituted or unsubstituted heteroaryl;

$R_{3a}$, $R_{3b}$ are each independently selected from the following group: a hydrogen, alkyl, halogen;

$R_{4a}$, $R_{4b}$ are each independently selected from the following group: a hydrogen, alkyl, halogen;

$R_5$ is selected from the following group: a hydrogen or alkoxy;

$R_6$ is selected from the following group: a hydrogen, substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, substituted or unsubstituted alkoxycarbonyl, substituted or unsubstituted carbamoyl, or $(CH_2)_tR_7$; wherein t is 1, 2, 3, 4, 5 or 6; wherein said $R_7$ is selected from the following group: a $C_2$-$C_6$ acyl, $C_2$-$C_6$ amido, aryl, heteroaryl, cycloalkyl, heterocycloalkyl, aryl$NR_8COR_9$; Said $R_8$ is selected from the following group: a hydrogen, alkyl; $R_9$ is selected from the following group: an alkyl, aryl, heteroaryl, cycloalkyl, heterocycloalkyl;

The A ring is a substituted or unsubstituted cycloalkyl;

n is 1, 2, 3, 4, 5, 6, 7, 8, 9 or 10;

Wherein each "substituted" means that a hydrogen atom on a group is replaced by one or more substituents selected from the group consisting of: a cyano, halogen, alkyl, hydroxy, alkoxy, alkenyl, alkynyl, aryl, heteroaryl;

and, said compound of formula I does not comprise

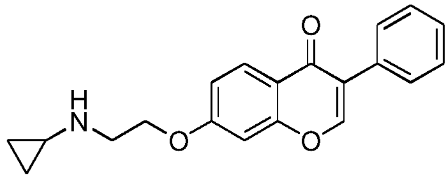
,
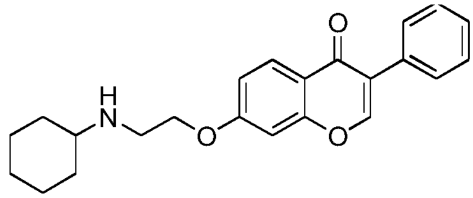
;

In the above formulae, unless otherwise specified, the alkyl group is a $C_1$-$C_6$ alkyl group of, the aryl group is a $C_6$-$C_{10}$ aryl group, the cycloalkyl group is a $C_3$-$C_8$ cycloalkyl group, the alkoxy group is a $C_1$-$C_6$ alkoxy group, the alkenyl group is a $C_2$-$C_6$ alkenyl group, the alkynyl group is a $C_2$-$C_6$ alkynyl group, and the heteroaryl group is a heteroaryl group of 5-12 members (more preferably, 5-7 members).

**2.** A compound of claim 1, wherein $X_1$ is O; and/or $X_2$ is O.

In another preferred example, said $R_7$ is selected from the following group: a hydrogen, alkyl.
In another preferred example, $X_3$ is O.
In another preferred example, $R_{1a}$, $R_{1b}$, $R_{1c}$ are each independently selected from a hydrogen.

**3.** A compound of claim 1, wherein $R_2$ is a substituted or unsubstituted aryl, preferably a substituted or unsubstituted phenyl.

In another preferred example, $R_{3a}$, $R_{3b}$ are each independently selected from the following group: a hydrogen, $C_1$-$C_4$ alkyl, halogen.
In another preferred example, $R_{4a}$, $R_{4b}$ are each independently selected from the following group: a hydrogen, $C_1$-$C_4$ alkyl, halogen.
In another preferred example, $R_5$ is selected from a hydrogen or $C_1$-$C_4$ alkoxy.
In another preferred example, $R_6$ is selected from the following group: a hydrogen, substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, or $(CH_2)_tR_7$; wherein t is 1, 2, 3, 4, 5 or 6; $R_7$ is selected from the following group: a $C_2$-$C_6$ acyl, $C_2$-$C_6$ amido, $NR_8COR_9$; Said $R_8$ is selected from the following group: a hydrogen, alkyl; $R_9$ is selected from the following group: an alkyl, aryl.
In another preferred example, the A ring is a substituted or unsubstituted $C_3$-$C_7$ cycloalkyl, preferably a $C_3$-$C_6$ cycloalkyl.
In another preferred example, n is 1, 2, 3 or 4.
In another preferred example, said $R_6$ is selected from the group consisting of: a hydrogen, substituted or unsubstituted alkyl.
In another preferred example, said $R_6$ is selected from the group consisting of: a hydrogen, unsubstituted alkyl.

**4.** A compound of claim 1, wherein said compound of formula I has the structure shown in formula II,

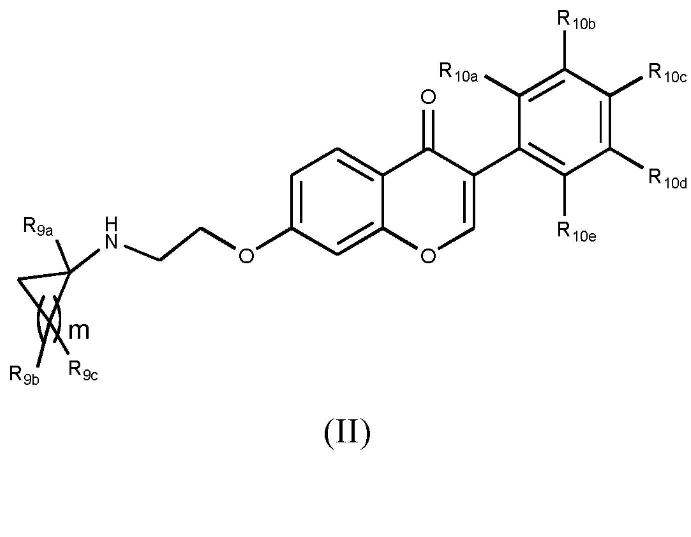

(II)

wherein $R_{9a}$, $R_{9b}$, $R_{9c}$ are each independently selected from the group consisting of: a hydrogen, hydroxyl, halogen, cyano, $C_1$-$C_6$ alkyl, $C_2$-$C_6$ alkenyl, $C_2$-$C_6$ alkynyl, $C_6$-$C_{10}$ aryl, 5-12 membered heteroaryl;

$R_{10a}$, $R_{10b}$, $R_{10c}$, $R_{10d}$, $R_{10e}$ are each independently selected from the group consisting of: a hydrogen, hydroxyl, $C_1$-$C_6$ alkoxy, halogen, cyano;
m is 1, 2, 3, 4 or 5.

**5.** A compound of claim 4, wherein the compound of formula II is selected from the following group:

| | |
|---|---|
| 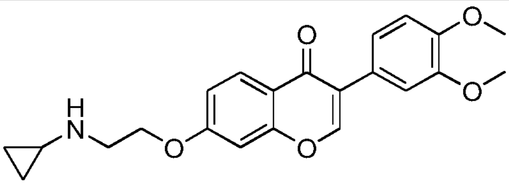 Compound 1 | 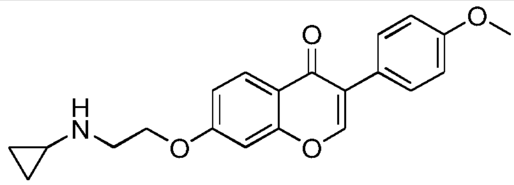 Compound 2 |
| 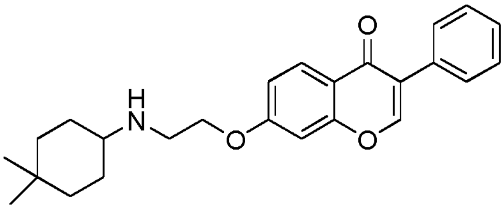 Compound 3 | 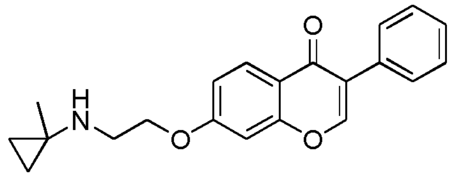 Compound 5 |
| 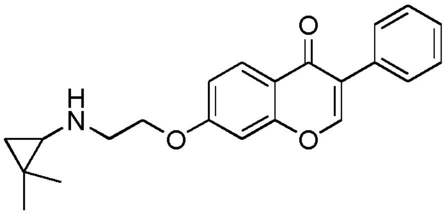 Compound 6 | 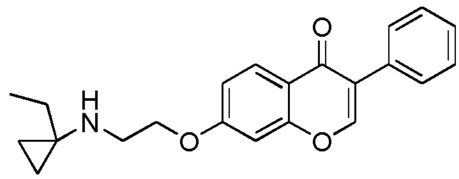 Compound 8 |

**6.** A compound of claim 1, wherein said compound of formula I has the structure shown in formula III,

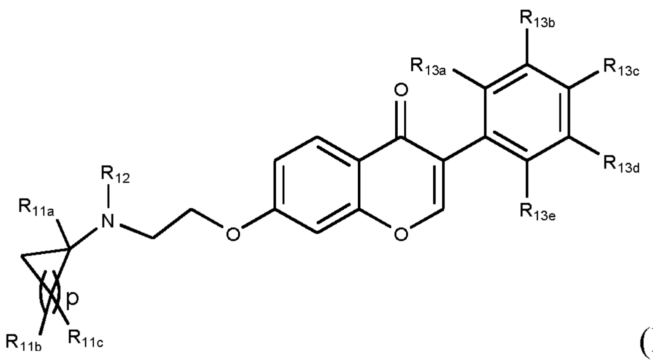

(III)

$R_{11a}$, $R_{11b}$, $R_{11c}$ are each independently selected from the group consisting of: a hydrogen, hydroxyl, halogen, cyano, C1-C6 alkyl, C2-C6 alkenyl, C2-C6 alkynyl, C1-C6 alkoxy C6-C10 aryl, and 5-12 membered heteroaryl;
$R_{12}$ is selected from the following group: a substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, substituted or unsubstituted alkoxycarbonyl, substituted or unsubstituted carbamoyl, or $(CH_2)_tR_7$; wherein t is 1, 2, 3, 4, 5, or 6; wherein said $R_7$ is selected from the group consisting of: a $C_2$-$C_6$ acyl, $C_2$-$C_6$ amido, aryl, heteroaryl, cycloalkyl, heterocycloalkyl, $NR_8COR_9$; wherein said $R_8$ is selected

from the group consisting of: a hydrogen, alkyl; and $R_9$ is selected from the group consisting of: an alkyl, aryl, heteroaryl, cycloalkyl, heterocycloalkyl;
$R_{13a}$, $R_{13b}$, $R_{13c}$, $R_{13d}$, $R_{13e}$ are each independently selected from the group consisting of: a hydrogen, hydroxyl, alkoxy, halogen, cyano.

**7.** A compound as claimed in claim 1, wherein the compound of formula III is selected from the following group:

| | |
|---|---|
| 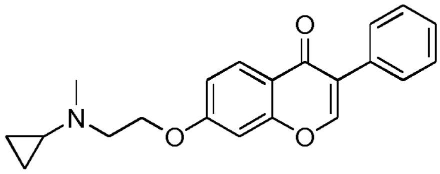<br>Compound 9 | 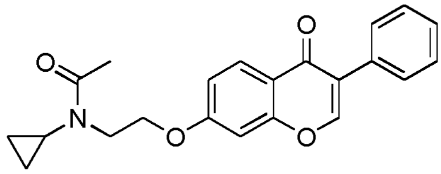<br>Compound 10 |
| 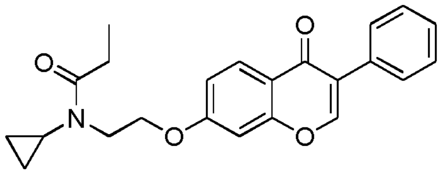<br>Compound 11 | 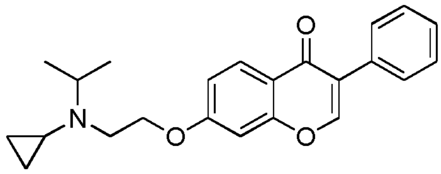<br>Compound 12 |
| 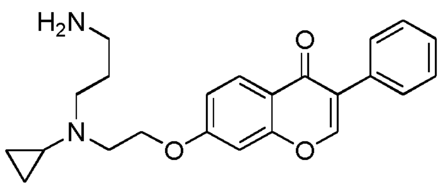<br>Compound 13 | 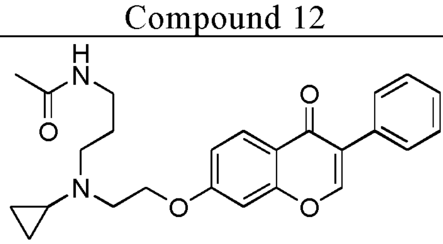<br>Compound 14 |

| | |
|---|---|
| 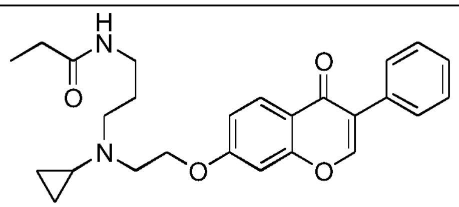<br>Compound 15 | 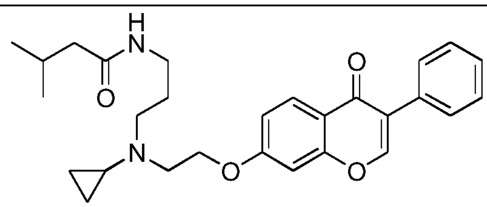<br>Compound 16 |
| 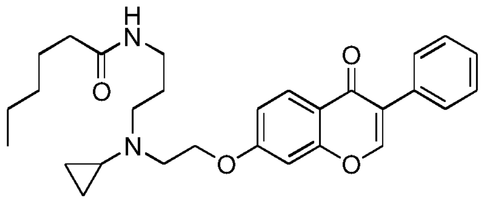<br>Compound 17 | 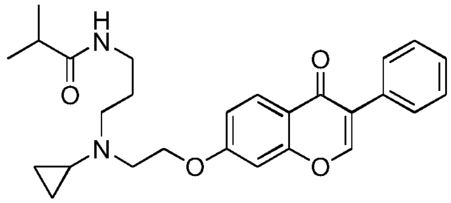<br>Compound 18 |
| 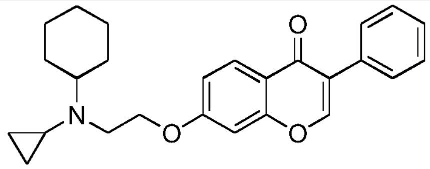<br>Compound 19 | 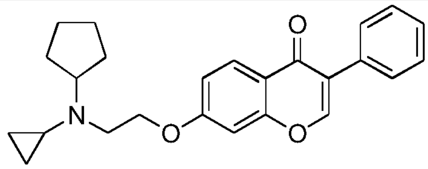<br>Compound 20 |
| 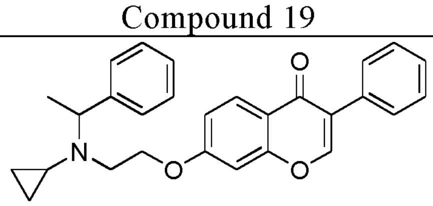<br>Compound 21 | 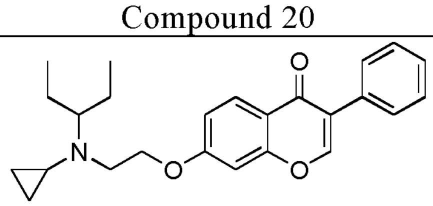<br>Compound 22 |

**8.** A pharmaceutical composition, wherein said said pharmaceutical composition comprising the compound of any one of claims 1-7,

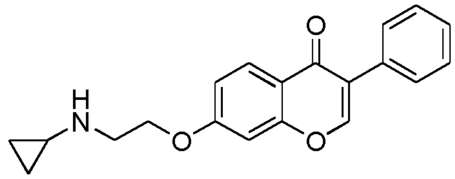

or

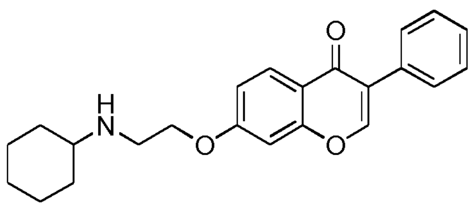

,

or a pharmaceutically acceptable salt, solvate, optically pure isomer or stereoisomer thereof; and a pharmaceutically acceptable carrier.

**9.** Use of a compound of formula I, or a pharmaceutically acceptable salt thereof, solvate, optically pure isomer,

stereoisomer, or mixture thereof, a pharmaceutical composition of claim8, for the preparation of a pharmaceutical composition;

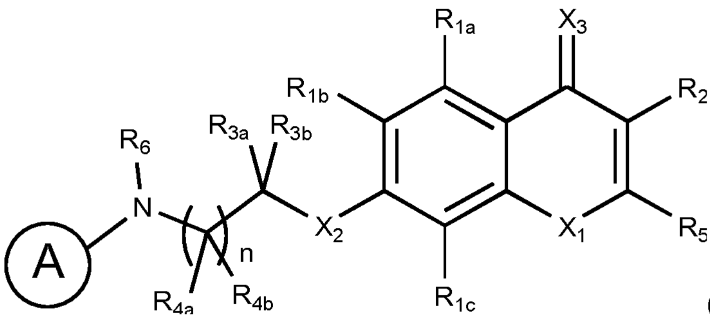

(I)

$X_1$, $X_2$ are each independently selected from the group consisting of: O, $NR_7$; wherein said $R_7$ is selected from the group consisting of: a hydrogen, alkyl, substituted alkyl, arylalkyl, substituted arylalkyl, cycloalkyl, substituted cycloalkyl;

$X_3$ is selected from the following group: O, $NR_8$; wherein said $R_8$ is selected from the following group: a hydrogen, alkyl;

$R_{1a}$, $R_{1b}$, $R_{1c}$ are each independently selected from the following group: a hydrogen, hydroxyl, alkoxy;

$R_2$ is selected from the following group: a substituted or unsubstituted aryl, or substituted or unsubstituted heteroaryl;

$R_{3a}$, $R_{3b}$ are each independently selected from the following group: a hydrogen, alkyl, halogen;

$R_{4a}$, $R_{4b}$ are each independently selected from the following group: a hydrogen, alkyl, halogen;

$R_5$ is selected from the following group: a hydrogen or alkoxy;

$R_6$ is selected from the following group: a hydrogen, substituted or unsubstituted alkyl, substituted or unsubstituted arylalkyl, aminoalkyl, substituted or unsubstituted cycloalkyl, substituted or unsubstituted heterocyclyl, substituted or unsubstituted $C_2$-$C_6$ acyl, substituted or unsubstituted alkoxycarbonyl, substituted or unsubstituted carbamoyl, or $(CH_2)_tR_7$; wherein t is 1, 2, 3, 4, 5 or 6; wherein said $R_7$ is selected from the following group: a $C_2$-$C_6$ acyl, $C_2$-$C_6$ amido, aryl, heteroaryl, cycloalkyl, heterocycloalkyl, aryl$NR_8COR_9$; Said $R_8$ is selected from the following group: 啊 hydrogen, alkyl; $R_9$ is selected from the following group: an alkyl, aryl, heteroaryl, cycloalkyl, heterocycloalkyl;

The A ring is a substituted or unsubstituted cycloalkyl;

n is 1, 2, 3, 4, 5, 6, 7, 8, 9 or 10;

Wherein each ""substituted" means that a hydrogen atom on a group is replaced by one or more substituents selected from the group consisting of: a cyano, halogen, alkyl, hydroxy, alkoxy, alkenyl, alkynyl, aryl, heteroaryl;

And the pharmaceutical composition is used for purposes selected from the following group:

(1) ameliorating, mitigating, or reversing a disease or condition caused by a viral infection;
(2) Acting as a SARS-Cov-2 viral ACE-2 receptor antagonist;
(3) As an inhibitor of post-infectious inflammatory cytokines.

**10.** The use of claim 9, wherein said virus is selected from the following group: influenza virus, respiratory syncytial virus, coronavirus (e.g., SARS-COV-2 virus), parainfluenza virus.

In another preferred example, said infection is a viral infection.

In another preferred example, said virus is selected from the following group: influenza virus, respiratory syncytial virus, coronavirus (e.g., SARS-COV-2 virus), parainfluenza virus.

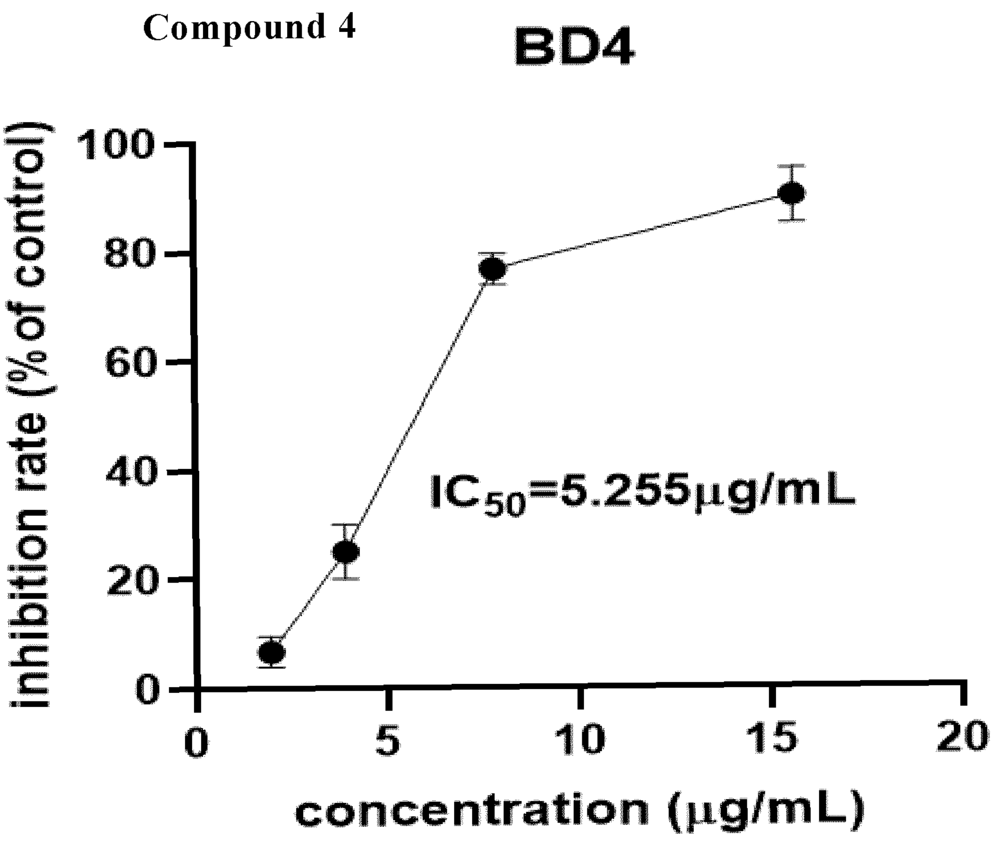
Compound 4
BD4
inhibition rate (% of control)
100
80
60
40
20
0
IC50=5.255μg/mL
0
5
10
15
20
concentration (μg/mL)

Fig. 1

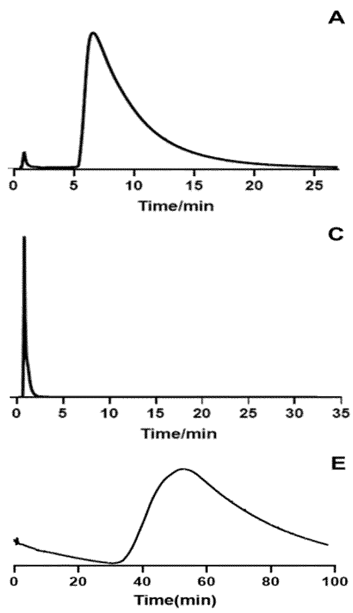


A: Compound 4; B: Compound F1; C: Compound F2; D: Compound F3; E: Chloroquine phosphate
KD values of the compounds of the present invention for their interaction with ACE2 receptors: Compound 4 has a relatively strong interaction with ACE2 receptors, and compound F1, compound F2, and compound F3 have no interaction with ACE2 receptors

Fig. 2

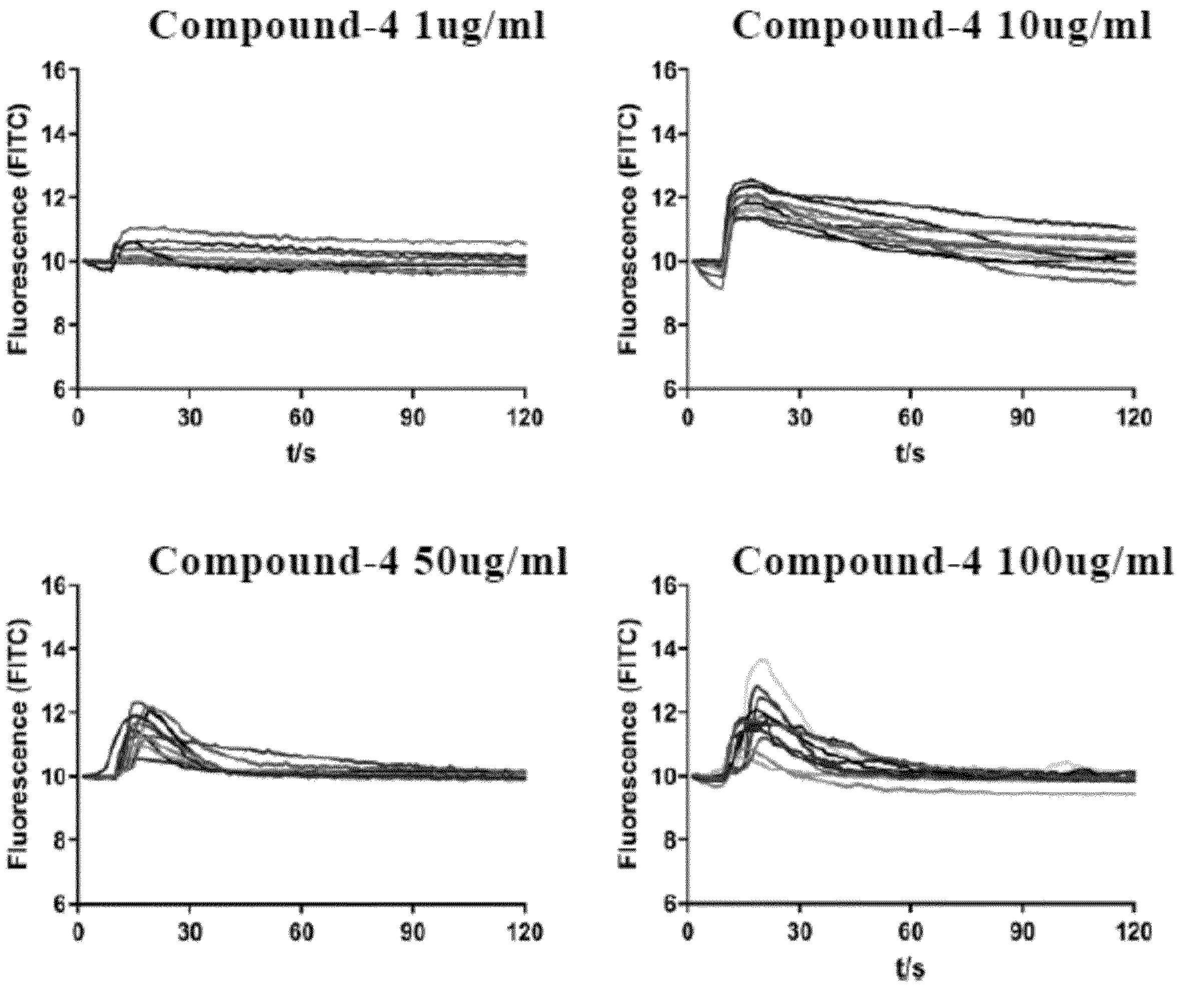
Compound-4 1ug/ml
Compound-4 10ug/ml
Compound-4 50ug/ml
Compound-4 100ug/ml
Fluorescence (FITC)
t/s
16
14
12
10
8
6
0
30
60
90
120

Fig. 3

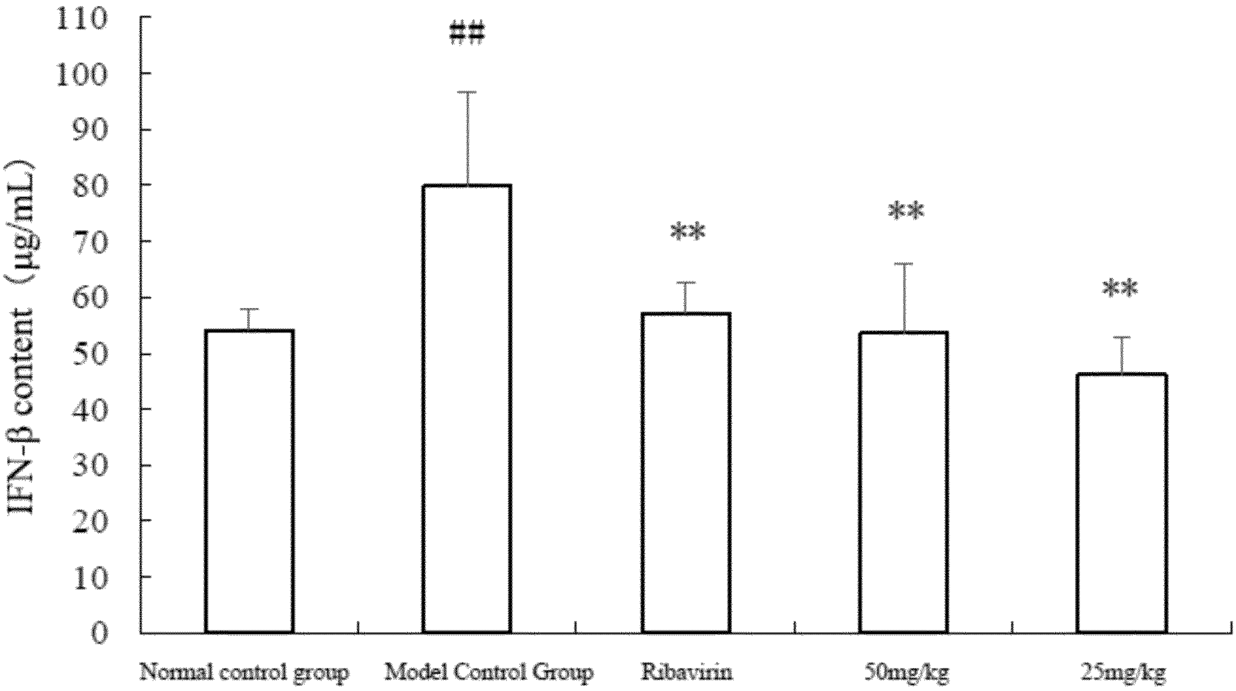
110
100
90
80
70
60
50
40
30
20
10
0
IFN-β content（μg/mL）
##
**
**
**
Normal control group
Model Control Group
Ribavirin
50mg/kg
25mg/kg

Fig. 4

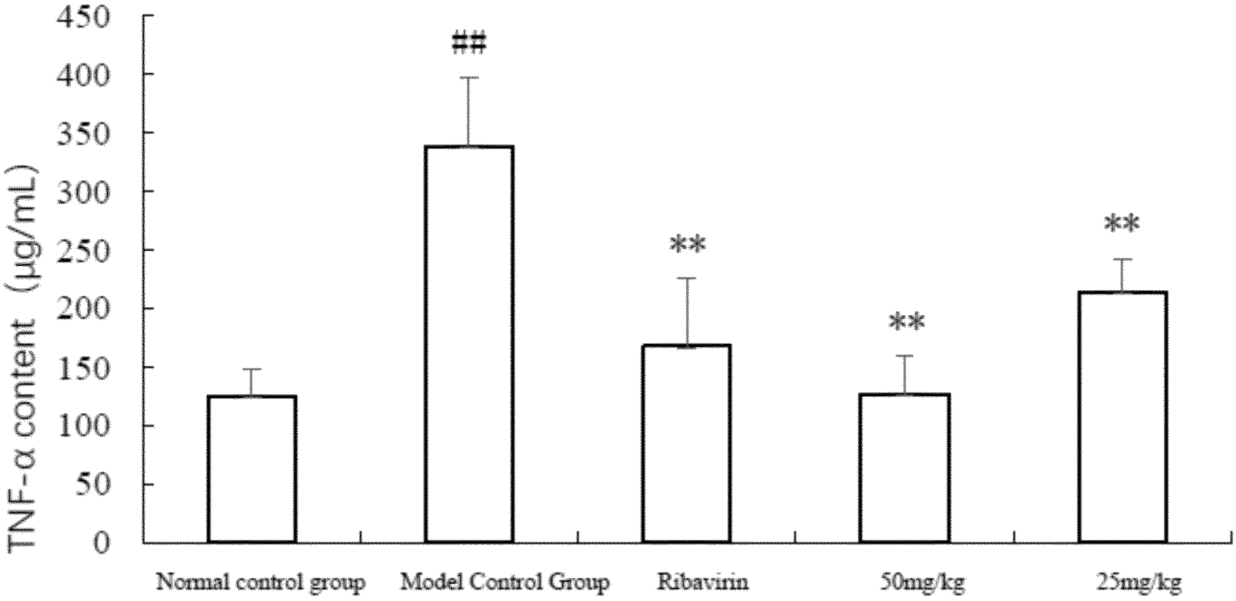
450
400
350
300
250
200
150
100
50
0
TNF-α content（μg/mL）
##
**
**
**
Normal control group
Model Control Group
Ribavirin
50mg/kg
25mg/kg

Fig. 5

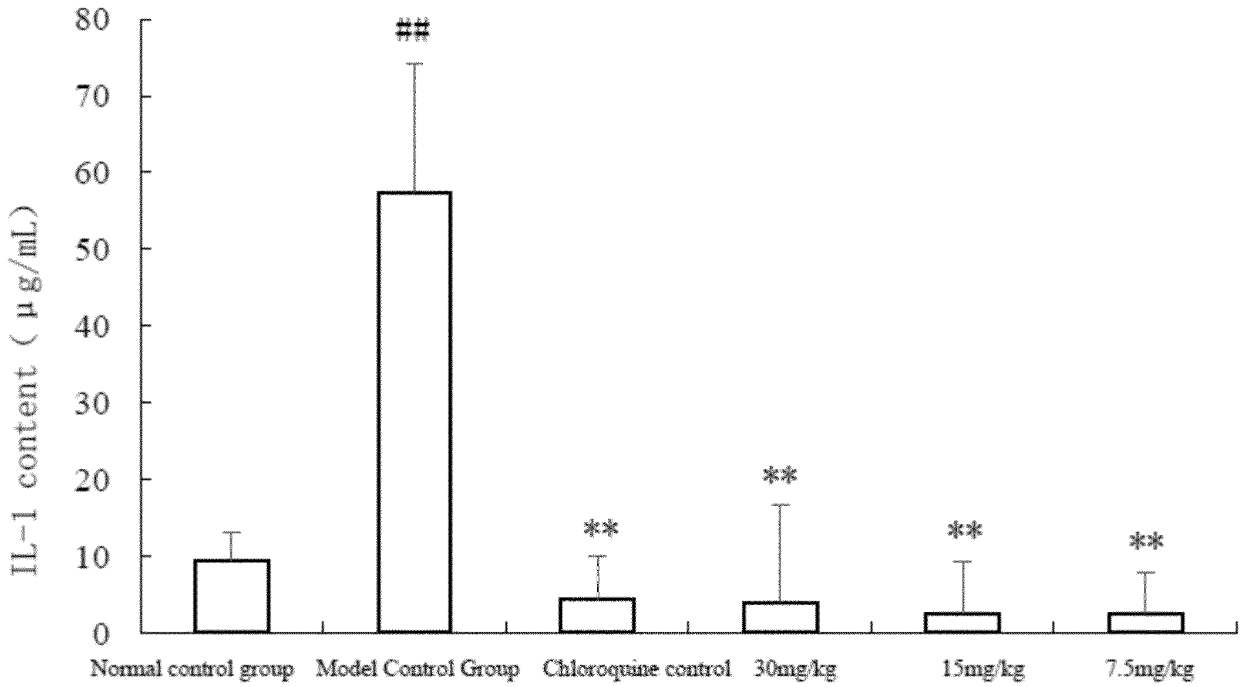
80
70
60
50
40
30
20
10
0
IL-1 content（μg/mL）
##
**
**
**
**
Normal control group
Model Control Group
Chloroquine control
30mg/kg
15mg/kg
7.5mg/kg

Fig. 6

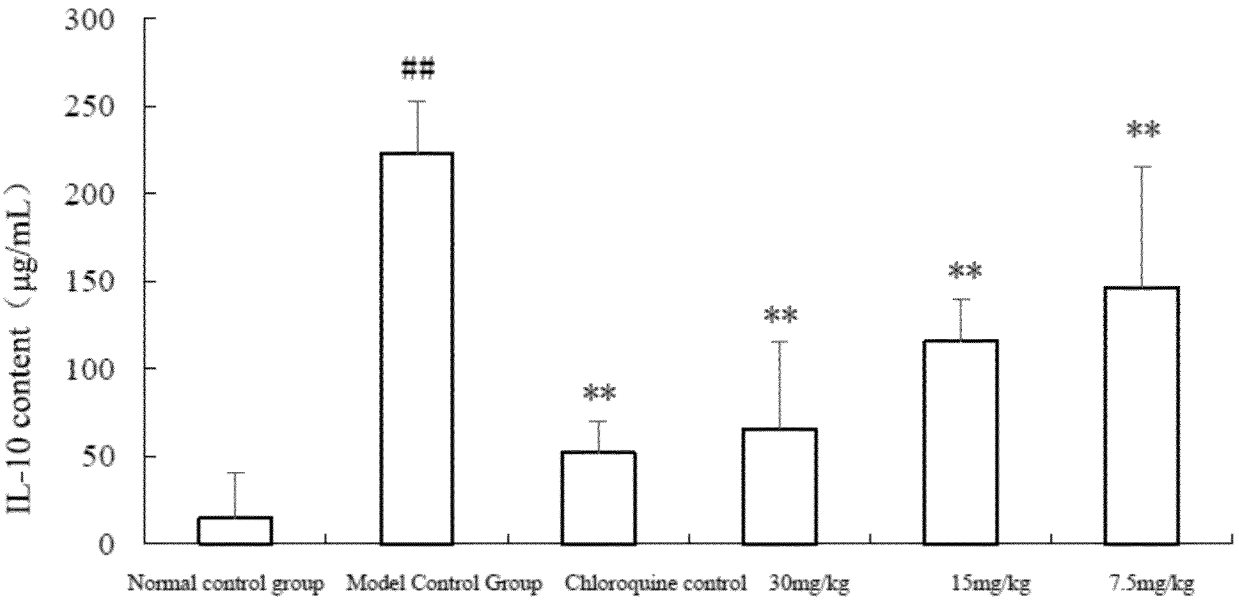
300
250
200
150
100
50
0
IL-10 content（μg/mL）
##
**
**
**
**
Normal control group
Model Control Group
Chloroquine control
30mg/kg
15mg/kg
7.5mg/kg

Fig. 7

**INTERNATIONAL SEARCH REPORT**

International application No.
**PCT/CN2023/073234**

**A. CLASSIFICATION OF SUBJECT MATTER**

C07D311/36(2006.01)i;A61K31/352(2006.01)i;A61P31/14(2006.01)i

According to International Patent Classification (IPC) or to both national classification and IPC

**B. FIELDS SEARCHED**

Minimum documentation searched (classification system followed by classification symbols)

IPC：C07D A61K A61P

Documentation searched other than minimum documentation to the extent that such documents are included in the fields searched

Electronic data base consulted during the international search (name of data base and, where practicable, search terms used)

CNABS, CNTXT, CNKI, 万方数据, WANFANG Data, VEN, ENTXT, CAplus, Registry, Marpat: 异黄酮, 色烯酮, 病毒, 流感, 冠状病毒, 副流感病毒, 呼吸道合胞病毒, isoflavone, Chromenone, virus, influenza, coronavirus, SARS+, parainfluenza, respiratory syncytial virus, structure search

**C. DOCUMENTS CONSIDERED TO BE RELEVANT**

| Category* | Citation of document, with indication, where appropriate, of the relevant passages | Relevant to claim No. |
|---|---|---|
| X | WANG, Dongmei et al. "Design, synthesis, and evaluation of isoflavone analogs as multifunctional agents for the treatment of Alzheimer's disease" *European Journal of Medicinal Chemistry,* Vol. 168, 20 February 2019 (2019-02-20), pp. 207-220 | 1-4 |
| X | CN 105377253 A (KINETA, INC.) 02 March 2016 (2016-03-02) see claims 1-23, and description, paragraphs 0089 and 0300 | 1-10 |
| X | WO 2016073947 A1 (KINETA, INC.) 12 May 2016 (2016-05-12) see description, paragraphs 0063-0065, 0099 and 0190, and claims 1-33 | 1-10 |
| A | CN 101723926 A (SICHUAN ACADEMY OF CHINESE MEDICINE SCIENCES) 09 June 2010 (2010-06-09) see entire document | 1-10 |
| A | CN 102617536 A (SHANGHAI RUIGUANG BIOCHEMICAL SCIENCE & TECHNOLOGY DEVELOPMENT CO., LTD. et al.) 01 August 2012 (2012-08-01) see entire document | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 April 2023** | **08 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/073234**

**C. DOCUMENTS CONSIDERED TO BE RELEVANT**

| Category* | Citation of document, with indication, where appropriate, of the relevant passages | Relevant to claim No. |
|---|---|---|
| A | US 2009215767 A1 (TZENG, CHERNG-CHYI et al.) 27 August 2009 (2009-08-27) see entire document | 1-10 |

**INTERNATIONAL SEARCH REPORT**
**Information on patent family members**

International application No.
**PCT/CN2023/073234**

| Patent document cited in search report | | | Publication date (day/month/year) | Patent family member(s) | | | Publication date (day/month/year) |
|---|---|---|---|---|---|---|---|
| CN | 105377253 | A | 02 March 2016 | AU | 2014290108 | A1 | 04 February 2016 |
| | | | | US | 2016122312 | A1 | 05 May 2016 |
| | | | | KR | 20160030940 | A | 21 March 2016 |
| | | | | CA | 2915874 | A1 | 22 January 2015 |
| | | | | JP | 2016528213 | A | 15 September 2016 |
| | | | | EP | 3021843 | A2 | 25 May 2016 |
| | | | | EP | 3021843 | A4 | 04 January 2017 |
| | | | | TW | 201542538 | A | 16 November 2015 |
| | | | | WO | 2015009812 | A2 | 22 January 2015 |
| | | | | WO | 2015009812 | A3 | 19 March 2015 |
| | | | | IL | 243608 | A0 | 29 February 2016 |
| WO | 2016073947 | A1 | 12 May 2016 | TW | 201625573 | A | 16 July 2016 |
| CN | 101723926 | A | 09 June 2010 | CN | 101723926 | B | 22 February 2008 |
| CN | 102617536 | A | 01 August 2012 | TW | 201542538 | A | 16 November 2015 |
| US | 2009215767 | A1 | 27 August 2009 | US | 7618998 | B2 | 17 November 2009 |
| | | | | TW | 200936126 | A | 01 September 2009 |
| | | | | TWI | 324514 | B | 11 May 2010 |

Form PCT/ISA/210 (patent family annex) (July 2022)